# EUROPEAN PATENT SPECIFICATION

(11) **EP 1 175 512 B1**
(45) Date of publication and mention of the grant of the patent: **20.02.2008**
(21) Application number: 00921163.2
(22) Date of filing: 10.04.2000
(51) Int. Cl.: C12Q 1/68

(54) **Method for the analysis of AFLP reaction mixtures using primer extension techniques**
Verfahren zur Analyse von AFLP Reaktionsmischungen unter Verwendung von Primer Verlängerungstechniken
Procède d'analyse de mélanges reactionells d'AFLP à l'aide de techniques d'extension d'amorce

(30) Priority: 09.04.1999 EP 99201112
(43) Date of publication of application: 30.01.2002
(73) Proprietor: KEYGENE N.V., 6700 AE Wageningen (NL)
(72) Inventor: VAN EIJK, Michiel, Josephus, Theresia, NL-3933 St Houten (NL); WITSENBOER, Hanneke, NL-6701 EC Wageningen (NL)
(74) Representative: van Westenbrugge, Andries
(86) International application number: PCT/NL2000/000234
(87) International publication number: WO 2000/061800

(56) References cited:
- WO-A-00/34518
- WO-A-90/09455
- WO-A-92/10587
- WO-A-93/25563
- WO-A-96/17082
- WO-A-96/22388
- WO-A-96/31622
- WO-A-97/27317
- WO-A-98/30721
- WO-A-98/44152
- WO-A-99/05321
- MCKENZIE STEVEN E ET AL: "Parallel molecular genetic analysis." EUROPEAN JOURNAL OF HUMAN GENETICS, vol. 6, no. 5, 1998, pages 417-429, XP000953253 ISSN: 1018-4813
- SYVANEN ANN-CHRISTINE: "From gels to chips: "minisequencing" primer extension for analysis of point mutations and single nucleotide polymorphisms." HUMAN MUTATION, vol. 13, no. 1, 6 April 1999 (1999-04-06), pages 1-10, XP000953256 ISSN: 1059-7794
- VOS P ET AL: "AFLP: A NEW TECHNIQUE FOR DNA FINGERPRINTING" NUCLEIC ACIDS RESEARCH,OXFORD UNIVERSITY PRESS, SURREY,GB, vol. 23, no. 21, 1995, pages 4407-4414, XP000939214 ISSN: 0305-1048

### SEQUENCE LISTING

<110> Keygene N.V.
<120> Method for the analysis of AFLP reaction mixtures using primer extension techniques
<130> BO 43583
<140> PCT
   <141> 2000-04-10
<160> 25
<170> PatentIn Ver. 2.1
<210> 1
   <211> 16
   <212> DNA
   <213> Artificial Sequence
<220>
   <223> Description of Artificial Sequence: primer
<400> 1
   gactgcgtac caattc 16
<210> 2
   <211> 16
   <212> DNA
   <213> Artificial Sequence
<220>
   <223> Description of Artificial Sequence: primer
<400> 2
   gatgagtcct gagtaa 16
<210> 3
   <211> 22
   <212> DNA
   <213> Artificial Sequence
<220>
   <223> Description of Artificial Sequence: primer
<400> 3
   agcagtagca accacttcag cc 22
<210> 4
   <211> 17
   <212> DNA
   <213> Artificial Sequence
<220>
   <223> Description of Artificial Sequence: primer
<400> 4
   atccggccag ttatacc 17
<210> 5
   <211> 22
   <212> DNA
   <213> Artificial Sequence
<220>
   <223> Description of Artificial Sequence: primer
<400> 5
   agcagtagca accacttcag cc 22
<210> 6
   <211> 17
   <212> DNA
   <213> Artificial Sequence
<220>
   <223> Description of Artificial Sequence: primer
<400> 6
   atccggccag ttatacc 17
<210> 7
   <211> 23
   <212> DNA
   <213> Artificial Sequence
<220>
   <223> Description of Artificial Sequence: primer
<400> 7
   tagcagtagc aaccacttca gcc 23
<210> 8
   <211> 16
   <212> DNA
   <213> Artificial Sequence
<220>
   <223> Description of Artificial Sequence: adapter
<400> 8
   gacgatgagt cctgag 16
<210> 9
   <211> 14
   <212> DNA
   <213> Artificial Sequence
<220>
   <223> Description of Artificial Sequence: adapter
<400> 9
   tactcaggac tcat 14
<210> 10
   <211> 16
   <212> DNA
   <213> Artificial Sequence
<220>
   <223> Description of Artificial Sequence: primer
<400> 10
   gatgagtcct gagtaa 16
<210> 11
   <211> 17
   <212> DNA
   <213> Artificial Sequence
<220>
   <223> Description of Artificial Sequence: primer
<400> 11
   gatgagtcct gagtaac 17
<210> 12
   <211> 19
   <212> DNA
   <213> Artificial Sequence
<220>
   <223> Description of Artificial Sequence: primer
<400> 12
   gatgagtcct gagtaacac 19
<210> 13
   <211> 19
   <212> DNA
   <213> Artificial Sequence
<220>
   <223> Description of Artificial Sequence: primer
<400> 13
   gatgagtcct gagtaacag 19
<210> 14
   <211> 17
   <212> DNA
   <213> Artificial Sequence
<220>
   <223> Description of Artificial Sequence: adapter
<400> 14
   ctcgtagact gcgtacc 17
<210> 15
   <211> 18
   <212> DNA
   <213> Artificial Sequence
<220>
   <223> Description of Artificial Sequence: adapter
<400> 15
   aattggtacg cagtctac 18
<210> 16
   <211> 19
   <212> DNA
   <213> Artificial Sequence
<220>
   <223> Description of Artificial Sequence: primer
<400> 16
   gtagactgcg taccaattc 19
<210> 17
   <211> 17
   <212> DNA
   <213> Artificial Sequence
<220>
   <223> Description of Artificial Sequence: primer
<400> 17
   gactgcgtac caattca 17
<210> 18
   <211> 18
   <212> DNA
   <213> Artificial Sequence
<220>
   <223> Description of Artificial Sequence: primer
<400> 18
   gactgcgtac caattcaa 18
<210> 19
   <211> 20
   <212> DNA
   <213> Artificial Sequence
<220>
   <223> Description of Artificial Sequence: oligonucleotide
<400> 19
   ttggctggca acgagcgaca 20
<210> 20
   <211> 21
   <212> DNA
   <213> Artificial Sequence
<220>
   <223> Description of Artificial Sequence: oligonucleotide
<400> 20
   tttcacccgc cggttagttt c 21
<210> 21
   <211> 21
   <212> DNA
   <213> Artificial Sequence
<220>
   <223> Description of Artificial Sequence: oligonucleotide
<400> 21
   tactgtccgc tctcgcattc a 21
<210> 22
   <211> 21
   <212> DNA
   <213> Artificial Sequence
<220>
   <223> Description of Artificial Sequence: oligonucleotide
<400> 22
   tgatcacgac atcacgttgc g 21
<210> 23
   <211> 21
   <212> DNA
   <213> Artificial Sequence
<220>
   <223> Description of Artificial Sequence: oligonucleotide
<400> 23
   tattgcgagc cacatcgttc c 21
<210> 24
   <211> 20
   <212> DNA
   <213> Artificial Sequence
<220>
   <223> Description of Artificial Sequence: oligonucleotide
<400> 24
   tggcctgaaa cgctgggttg 20
<210> 25
   <211> 21
   <212> DNA
   <213> Artificial Sequence
<220>
   <223> Description of Artificial Sequence: oligonucleotide
<400> 25
   tttttctcgg cttttctttc t 21

## Description

The present invention relates to a method for analysing nucleic acid sequences and to an array for use in such a method.

In particular, the invention relates to arrays and methods for determining whether a specific nucleic acid sequence is present or absent in a nucleic acid sequence or mixture of nucleic acid sequences.

More in particular, the invention relates to a method and array for determining the presence or absence, in genomic DNA or a sample of restriction fragments derived from genomic DNA, of sequences that correspond to unique restriction fragments that can serve as genetic markers, such as AFLP-markers.

The method and arrays of the invention are in particular suited for the analysis of amplified mixtures of restriction fragments, such as reaction mixtures resulting from AFLP.

A number of methods for analyzing nucleic acid sequences are known. In general, these methods comprise immobilization of the sequences to be analysed, for instance by blotting; hybridization of the sequences with a labeled DNA- or RNA-probe; stringency washes to remove non-hybridized material; followed by detection of those sequences that have hybridized with the probe.

Such techniques are sometimes carried out after prior amplification -such as by PCR- of the starting nucleic acid sequences, usually a mixture of restriction fragments from a genomic DNA. The resulting mixture of amplified fragments is then separated, for instance on the basis of differences in length or molecular weight, such as by gel-electrophoresis, and then visualised, i.e. by blotting followed by hybridization. The resulting pattern of bands is referred to as a DNA fingerprint.

Usually in DNA fingerprinting, fingerprints of closely related species, subspecies, varieties, cultivars, races or individuals are compared. Such related fingerprints can be identical or very similar, i.e. contain a large number of corresponding -and therefore less informative- bands.

Differences between two related fingerprints are referred to as "DNA polymorphisms". These are DNA fragments (i.e. bands) which are unique in or for a specific fingerprint. The presence or absence of such polymorphic bands, or the pattern thereof, can be used as a genetic marker, i.e. to identify a specific species, subspecies, variety, cultivar, race or individual, to establish the presence or absence of a specific inheritable trait, of a gene, or to determine the state of a disease.

For a further discussion and definitions of DNA-fingerprinting, DNA typing, DNA polymorphisms, genotyping, PCR and similar techniques, reference is made to the discussion of the prior art in EP-0 534 858 A1.

The art also describes oligonucleotide arrays for analysing nucleic acid sequences or mixtures thereof, vide for instance WO 97/27317, WO 97/22720, WO 97/43450, EP 0 799 897, EP 0 785 280, WO 97/31256, WO 97/27317 and WO 98/08083. WO9325563 describes an allele specific primer extension method. WO9631622 describes a method for detecting mutations, whereby target sequences are incubated with an oligonucleotide probe on a solid support to form a duplex. WO9905321 discloses the use of solid substrates for performing amplification or enzymatic reactions thereon.

WO 90/09455, WO 91/02087, WO 91/13075, WO 92/15712 and EP 0 123 513 all describe techniques for detecting point mutations at a predetermined site of a DNA sequence (usually full length genomic DNA or cDNA), which are generally referred to as "minisequencing". See also Syvänen, 1999 (Human Mutation 13:1-10).

Minisequencing is based upon extension of a primer that hybridizes with part of the DNA sequence such that the 3' end of the primer is immediately adjacent to the point mutation. The hybrid thus obtained is contacted - usually in a single "one-tube" reaction step - with a mixture containing at least one detectable nucleotide, under conditions that extension of the primer with the detectable nucleotide takes place when the detectable nucleotide is complementary to the nucleotide present at the site of the point mutation, but no extension takes place when the detectable nucleotide does not correspond to the nucleotide present at the point mutation, so that whether or not extension of the primer takes place provides information on the nucleotide present at the site of the point mutation. (Alternatively, 2-4 differently labeled nucleotides can be used simultaneously, the extension with a specific labeled nucleotide being indicative for the presence of a complementary nucleotide at the site of mutation).

Besides being provided with a detectable functionality, the detectable nucleotide(s) and reaction mixtures/conditions used in mini-sequencing are also chosen such that, after the labeled nucleotide has been added to the primer, no further extension of the primer takes place ("terminator mixtures"). For instance, chain-terminating dideoxyribonucleoside triphosphates (ddNTPs) or thionucleotides can be used.

The method described differs from minisequencing at least in the following, non-limiting aspects:
a) Minisequencing is used to detect/determine point mutations at a specific, known site in the genome, and is not used to determine the presence of absence of polymorphic fragments in a mixture of (amplified) restriction fragments;
b) Partly as a consequence of a), in minisequencing, (amplified) total genomic or cDNA is used as the starting material, not a mixture of (amplified) restriction fragments;
c) in minisequencing, usually only one or at most a small number of mutations are investigated simultaneously; in the invention, a mixture of (amplified) restriction fragments will usually be tested for the presence of at least 100 to more 1000 markers simultaneously;
d) in minisequencing, it is generally difficult to generate template DNA in multiplex form;
e) partly as a consequence of c) and d), in minisequencing, arrays containg a large number of different primers will not be used.

Selective restriction fragment amplification or AFLP, a DNA-fingerprinting technique which requires no prior knowledge of the sequence to be analysed, is described in the European patent application 0 534 858 by applicant and by Vos et al., 1995 (Nucleic Acid Research Vol 23., No. 21: 4407-4414). This technique generally comprises the steps of:
(a) digesting a nucleic acid, in particular a DNA, with one or more specific restriction endonucleases, to fragment said DNA into a corresponding series of restriction fragments;
(b) ligating the restriction fragments thus obtained with at least one double-stranded synthetic oligonucleotide adapter, one end of which is compatible with one or both of the ends of the restriction fragments, to thereby produce tagged restriction fragments of the starting DNA;
(c) contacting said tagged restriction fragments under hybridizing conditions with at least one oligonucleotide primer,
(d) amplifying said tagged restriction fragment hybridized with said primers by PCR or a similar technique so as to cause further elongation of the hybridized primers along the restriction fragments of the starting DNA to which said primers hybridized; and
(e) identifying or recovering the amplified or elongated DNA fragment thus obtained.

The thus amplified DNA-fragments can then be analysed and/or visualised, for instance by means of gel-electrophoresis, to provide a genetic fingerprint showing bands corresponding to those restriction fragments that have been linked to the adapter, recognized by the primer, and therefore amplified during the amplification step; the resulting bands providing information on the specific restriction site pattern of the starting DNA.

By comparing AFLP-fingerprints from related individuals, bands which are unique for each fingerprint can be identified. These polymorfisms are referred to as "AFLP-markers", and can again be used to identify a specific individual, cultivar, race, variety, subspecies or species, and/or to establish the presence or absence of a specific inherited trait, gene or disease state.

For a further description of AFLP, its advantages, its embodiments, as well as the techniques, enzymes, adapters, primers and further compounds and tools used therein, reference is made to EP-A-0 534 858 and co-pending European applications EP 0 976 835 and EP 0 974 672 all by applicant. Also, in the description hereinbelow, the definitions given in paragraph 5.1 of EP-0 534 858 will be used, unless indicated otherwise.

Although AFLP is generally less time-consuming than hybridisation-based techniques, it still suffers from the disadvantage that the amplified fragments have to be separated (i.e. by gel-electrophoresis) and visualized (i.e. by generation of a fingerprint). These are very elaborate and time consuming procedures, which require special apparatus, such as electrophoresis and auto-radiography equipment. Thereafter, the fingerprints have to be analysed -nowadays generally performed by "reading" the fingerprint into a computer- to identify the polymorphic bands.

However, such detection by polyacrylamide gelelectrophoresis may be a limiting factor of the enormous multiplex capacity of the AFLP-technology for high throughput marker detection.
WO98/30721 discloses a method based on AFLP, whereby the need for PAGE is rendered superfluous by testing the amplified sample for its ability to hybridize with an AFLP-generated polynucleotide probe.

Therefore, a first aim of the invention is to provide an alternative technique to WO98/30721 for analysing nucleic acid sequences - in particular for determining the presence or absence of AFLP markers - which no longer requires the use of gel-electrophoresis and preferably also avoids the use of autoradiography and/or of radioactive materials, and thereby improves throughput (and provides a method for high-throughput AFLP marker detection).

This is achieved by the method of the invention, which is based upon specific extension/elongation of a oligonucleotide sequence (i.e. primer) that is complementary to (part of) the fragment to be detected, such that extension of the complementary oligonucleotide will only take place when the fragment to be detected is present, and will not take place when the fragment to be detected is absent. Thus, whether or not the oligonucleotide is extended will be indicative for the presence or absence (respectively) of the fragment to be detected.

This extension-based detection can be used instead of getectrophoresis/autoradiography, in analysing AFLP reaction mixtures, in particular for routine, high throughput genotyping. For this purpose, the invention *inter alia* also provides an array of oligonucleotides that can be used to test an AFLP reaction mixture for the presence of several AFLP markers simultaneously, i.e. in a single detection step.

In a first aspect, the invention therefore relates to a method for determining the presence or absence of a target restriction fragment in a mixture of restriction fragments, using an oligonucleotide sequence that is essentially complementary to part of the target restriction fragment, said method comprising the steps of:
a) contacting the mixture of restriction fragments with the oligonucleotide sequence under hybridization conditions, such that when the target restriction fragment is present, a hybrid is formed between the target restriction fragment and the oligonucleotide sequence, such that resulting hybrid has at least one unpaired nucleotide of the target restriction fragment directly adjacent to the 3' end of the oligonucleotide sequence;
b) adding at least one labelled nucleotide or nucleotide analogue to the mixture resulting from step a), under conditions suitable for extension of an oligonucleotide; such that when a hybrid of the target restriction fragment and the oligonucleotide is present, and said at least one labeled nucleotide or nucleotide analog is complementary to the at least one unpaired nucleotide of said target restriction fragment directly adjacent to the 3' end of the oligonucleotide sequence, the nucleotide sequence is extended with the labeled nucleotide or nucleotide analog;
c) detecting the presence or absence of any hybrid with an added labeled nucleotide or nucleotide analog, and/or of any oligonucleotide sequence with an added labeled nucleotide or nucleotide analog.

The method of the invention may further contain one or more steps in which the hybrid formed between the target restriction fragment and the oligonucleotide sequence is separated from any restriction fragments not hybridized to a oligonucleotide sequence, as well as any other unwanted sequences or compounds. Such a step may be carried out after step a), after step b), or both.

Furthermore, it will be clear to the skilled person that the order in which the various compounds/sequences (i.e. the restriction fragments, the oligonucleotide and the labeled nucleotide) are added to/ mixed with each another in steps a) and b) may be varied, and such variations will fall within the scope of the invention and claims. However, the order decribed above represents the most convenient way of carrying out the invention.

The invention further relates to a method for determining the presence or absence of one or more target restriction fragments in a mixture of restriction fragments, comprising the steps of:
a) contacting the mixture of restriction fragments under hybridizing conditions with at least one oligonucleotide sequence, said oligonucleotide sequence being complementary to part of a target restriction fragment, but not to any other restriction fragment in the mixture, such that the resulting hybrid has at least one unpaired nucleotide of said target restriction fragment directly adjacent to the 3' end of the oligonucleotide sequence;
b) extending the oligonucleotide sequence with at least one labeled nucleotide or nucleotide analog, said at least one labeled nucleotide or nucleotide analog being complementary to the at least one unpaired nucleotide of said target restriction fragment directly adjacent to the 3' end of the oligonucleotide sequence;
c) detecting the oligonucleotide sequence with the added labeled nucleotide or nucleotide analog.

Again this method can contain one or two optional steps for separating the target restriction fragments hybridized with the oligonucleotide sequence from any restriction fragments not hybridized to a oligonucleotide sequence; as well as other unwanted sequences and excess reagents.

In the method of the invention, during step a), the mixture of restriction fragments will usually be contacted with simultaneously with at least 3, preferably at least 10, more preferably at least 50, most preferably at least 100 different oligonucleotide sequences, wherein each oligonucleotide sequence is most preferably specific for only one target restriction fragment. For this purpose, in the method of the invention, the oligonucleotide sequence(s) used preferably are bound to a solid support, more preferably so as to form an array, and such arrays form a further aspect of the invention.

By "an oligonucleotide specific for a target restriction fragment" is meant that the oligonucleotide sequence is essentially complementary only to the intended target restriction fragment, but most preferably not essentially complementary to any other restriction fragment in the mixture. An oligonucleotide sequence is considered "essentially complementary to" a target restriction fragment when it has a high degree of sequence homology with the corresponding part of the target restriction fragment (determined on the basis of the full length of the oligonucleotide sequence), i.e. of at least 90%, preferably at least 95%, most preferably at least 99%.

In the present description, the restriction fragment to be detected is referred to as the *"Target Sequence".*

Generally, a Target Sequence will be characterized in that it is obtainable/obtained by cutting a starting DNA, usually a genomic DNA or cDNA, with at least one, but commonly with at least two restriction enzymes, of which preferably at least one is a "frequent cutter" restriction enzyme and at least one is a "rare cutter" restriction enzyme. (In case of genomic DNA, the "frequent cutter" serves the purpose of reducing the size of the restriction fragments to a range of sizes which are amplified efficiently and in a manner compatible with the detection technique used, and the "rare cutter" serves the purpose of controlling the total number of fragments generated. For both, reference is made to EP-A-0 534 858 and EP-A-0 721 987 by applicant; Non-limiting examples of suitable frequent cutter enzymes include *Mse*I and *Taq*I. Non-limiting examples of commercially available rare cutters include *Pst*I*, Hpa*II*, Msp*I*, Cla*I*, Hha*I*, EcoR*I*, EcoR*II*, BstB*I*, HinP*1*, Hin*DIII*, Mae*II*, Bbv*I*, Pvu*II*, Xma*I*, Sma*I*, Nci*I*, Ava*I*, Hae*II*, Sal*I*, Xho*I*, Bst*YI*, Bam*HI*, Bgl*II and *Pvu*II*,* of which *Pst*I*, Hpa*II*, Msp*I*, Cla*I*, EcoR*I*, EcoR*II*, Bst*BI*, Hin*P1*, Hin*DIII, *Bam*HI, *Bgl*II and *Mae*II are preferred.

Preferably, the Target Sequence is a restriction fragment as present in a mixture of restriction fragments, more preferably an amplified restriction fragment as present in a mixture of restriction fragments and/or amplified restriction fragments.

Even more preferably, the Target Sequence is a restriction fragment that corresponds to a polymorphic fragment or band of interest, such as an AFLP-marker. As such, the Target Sequence may be a non-amplified fragment present in a mixture of restricted DNA, or may be a restriction fragment amplified by AFLP as present in reaction mixture obtained after AFLP amplification.

The oligonucleotide sequence used to detect the Target Sequence will be referred to hereinbelow as the *"Detection Oligonucleotide",* the *"Detection Sequence"* or the *"Detection Primer",* which terms are to be considered equivalent.

The Detection Sequences may further contain a "tail" -such as a polyT sequence- to improve accessability for the Target Sequence.

Each Detection Sequence should at least in part be complementary to a specific Target Sequence, as defined above. The Detection Sequence may be any nucleic acid (i.e. DNA or RNA) but is preferably DNA. The Detection Sequence will generally have a size of about 10 to 100 base pairs, preferably about 20 to 50 base pairs. The Detection Sequences may all be of the same size, or may be of different sizes. The Detection Sequence can be obtained in any suitable manner. For instance, when one or more polymorphic bands have been identified in an AFLP fingerprint of a specific set of (preferably related) individuals, the sequence of each band/fragment may be determined in a manner known per se, and Detection Sequences may be synthesized that are complementary to any part of the sequence of each of the polymorphic bands, i.e. using an automated DNA-synthesizer or in any other manner known per se. Also, solid phase nucleic acid synthesis techniques may be used, which may result directly in an array with the desired Detection Sequences, as described below. Furthermore, the Detection Sequence may be obtained using known techniques of genetic engineering, for instance by primer extension using the Target Sequence as a template, and/or by using one or more restriction enzymes, optionally using amplification.

Also, the Detection Sequence may obtain one or more "alternative nucleosides" as described in applicants copending European application EP 0 974 672, so that the Detection Sequence is an "alternative primer" as described therein. Similarly, in step b), the Detection Sequence may be extended with such an alternative nucleoside/nucleotide, provided with a label. Examples thereof include the bases Inosine (I) and Uracil (U), as well as dUTP and dITP, and these are included within the term "labeled nucleotide analog" as mentioned above. It is to be understood that the presence of such alternative nucleosides does not prevent the Detection Sequence and the Target Sequence to be essentially complementary to one another as defined above.

When the mixture of restriction fragments to be investigated for the presence of one or more target sequences has been amplified using AFLP, (part of) the Detection Sequence may be complementary to (part of) the original restriction fragment, to (part of) the adapter sequence, or both. According to one preferred embodiment, the Detection Sequence corresponds to an AFLP-primer with one or more (further) selective nucleotides added to the 3' end. More preferably, the Detection Sequence corresponds to (one of) the AFLP primer(s) used to amplify the restriction fragments, with one or more (further) selective nucleotides added to the 3' end.

For instance, when the restriction fragments have been amplified using a +(n) AFLP primer, one or more +(n+q) primers may be used as a Detection Sequence, which incorporate (part of) the constant region of the original +(n) AFLP primer as well as the (n) selective nucleotides of the original primer, with (q) further nucleotide(s) added at the 3' end (n usually being 0-6, q usually being 1-10).

Preferably, such a Detection Sequence based on an AFLP primer contains a total 5-15, preferably 7-12 selective nucleotides, i.e. at the 3' end of the sequence that corresponds to the constant region of the AFLP primer. Especially suited combinations of AFLP-primer and Detection Sequence are a +4 to +6 AFLP primer for the amplification, combined with a corresponding +7 to + 12 Detection Sequence in step b) of the invention. Already good results (i.e. sensitivity and selectivily) can be obtained using a +6 AFLP primer in combination with a corresponding +7 Detection Sequence.

As mentioned above, the Detection Sequences are preferably bound to a solid support, more preferably so as to form an array. Such an array will generally comprise at least 10, more specifically at least 100, more preferably at least 1000 different Detection Sequences. For a "high-density array" or "micro-array", the total number of Detection Sequences can be in the range of 1000- 100.000 per cm² of surface area.

The Detection Sequences will generally be bound to the carrier in such a way that each Detection Sequence is attached to, and corresponds with, a specific, distinct part of the carrier, so as to form an independently detectable area on the carrier, such as a spot or band. This makes it possible to "read" the array by scanning (i.e. visually or otherwise) the areas to which each Detection Sequence (i.e. a sequence corresponding to a marker of interest) is attached.

Preferably, the Detection Sequences are bound to the carrier in accordance with a predetermined, regularly distributed pattern, in which for instance related Detection Sequences (i.e. corresponding to related markers) can be grouped together, i.e. in one or more lines, columns, rows, squares, rectangles, etc, preferably in an "adressable" form. This further facilitates analysis of the array.

The density of the different Detection Sequences will generally be in the range of 1-100,000 different Detection Sequences/cm², usually 5-50,000 Detection Sequences/cm², generally between 10-10,000 Detection Sequences/cm².

An array of the invention can (also) contain sets of Detection Sequences that correspond to markers indicative for different (i.e. genetically unrelated) traits or properties, and such an array can be used to analyse an individual (genome) for the presence or absence of all these properties simultaneously. However, the Detection Sequences will usually correspond to markers from within one "genotyping collection", i.e. from markers as may be present in individuals that belong to the same family, genus or preferably species, i.e. so as to provide -for instance- a "maize-array", a "tomato-array", a "wheat-array" etc..

In one embodiment, the Detection Sequences present on the array will correspond to AFLP-markers that are representative of different subspecies, varieties, cultivars, lines or races of the same species.

An array of the invention can also contain Detection Sequences that correspond to markers that are representative of a certain genetic state of an individual, such as the presence or absence of a disease state, i.e. of oncogenes and of genetically determined diseases.

Although preferably each Detection Sequence on the array will correspond to a polymorphic band of interest (i.e. a marker), the presence on the array of some non- or less informative Detection Sequences (for instance corresponding to non-polymorphic bands or to markers that are too abundant to provide useful information) is not excluded. However, these will usually constitute less than 50%, preferably less than 30%, more preferably less than 10% of all Detection Sequences present on the array. It is also included that some or most of the Detection Sequences may be informative for one specific application or genome, but not for another. However, preferably 95-100% of all Detection Sequences will correspond to or be representative of an AFLP-marker.

The solid support (i.e. carrier) for the array may be any solid material to which nucleic acid sequences can be attached, including porous, fibrous, woven and non-woven materials, as well as composite materials. Also, semi-solid materials such as gels or matrices (for instance as used in chromatography) may be used, although this is not preferred.

Suitable carriers include, but are not limited to, those made of plastics, resins, polysaccharides, silica or silica-based materials, functionalized glass, modified silicon, carbon, metals, inorganic glasses, membranes, nylon, natural fibers such as silk, wool and cotton, and polymer materials such as polystyrene, polyethylene glycol tetra-phthalate, polyvinyl acetate, polyvinyl chloride, polyvinyl pyrrolidone, polyacrylonitrile, polymethyl methacrylate, polytetrafluoroethylene, butyl rubber, styrenebutadiene rubber, natural rubber, polyethylene, polypropylene, (poly)tetrafluoroethylene, (poly)vinylidenefluoride, polycarbonate and polymethylpentene. Further suitable support materials are mentioned for instance mentioned in US-A-5,427,779, WO 97/22720, WO 97/43450, WO 97/31256, WO 97/27317 and EP 0 799 897.

Preferably, the carrier will have an essentially flat, rectangular shape, with the Detection Sequences bound to one surface thereof. However, any other suitable two- or three-dimensional form may also be used, such as a disc, a sphere or beads, or materials or structures that allow a liquid medium containing the sample to be analysed to pass or flow through the carrier, such as columns, tubes or capillairies, as well as (macro)porous- , web- or membrane-type structures, including the flow-through genosensor devices referred to in WO 97/22720.

The size of the array, as well as of the individual areas corresponding to each of the different Detection Sequences, may vary, depending upon the total amount of Detection Sequences, as well as the intended method for analysing the array.

For an array that is to be inspected visually, the total array and the separate areas thereon may be of such a size that they can be seen and distinguished with the naked eye or through a microscope, i.e. in the range of 1 to 12 cm² for the total array, and 0.01 -1 cm² for the individual areas.

Arrays that are analysed using other types of (usually automated) scanning equipment may be of smaller size, and are preferably in the form of high-density or micro-arrays, in the range of 0.01 -12 cm² for the total array, and 0.0001 - 1 cm² for the individual areas. This allows hybridization to be carried out in a small volume on a small sample, or even the use of flow-through techniques.

The Detection Sequences may be bound to the carrier in any manner known per se, and the specific technique used will mainly depend upon the carrier used. Binding will be at the 5'-end, or somewhere else on the Detection Sequences, as appropriate, but preferably not at the 3'-end, as this is to be extended during the primer extension reaction.

Preferably, the Detection Sequences will be covalently bonded to the array, i.e. by a suitable chemical technique. For this purpose, the Detection Sequences and/or the carrier may be modified to carry one or more groups or functionalities for attaching the Detection Sequence to the array. For instance, the surface of the carrier may be activated to carry one or more groups such as carboxy, amino, hydroxy, etc..

Suitable methods for attaching the Detection Sequences to the carrier will be clear to the skilled person. In general, any method for attaching a nucleic acid to a solid support can be used, including the methods described in US-A-5,427,779; US-A-4,973,493; US-A-4,979,959; US-A-5,002,582; US-A-5,217,492; US-A-5,525,041; US-A-5,263,992; WO 97/46313 and WO 97/22720, as well as the references cited therein.

As an example of covalent attachment, coupling can proceed using photoreactive groups such as N-oxy-succinimide, in which either the array-bound Nucleic Acid Sequence is derivatized with a photoreactive group and attached to the surface, or the surface is first treated with a photoreactive group, followed by application of the Detection Sequence, for instance in N-terminal amino-modified form. A suitable protocol, following the general method described in Amos et al., Surface Modification of Polymers by Photochemical Immobilization, The 17th Annual Meeting of the Society of Biomaterials, May 1991, Scottsdale AZ, in given in WO 97/46313.

Other covalent binding techniques involve the use of 3'-aminopropanol-groups or epoxysilane-amine chemistry, for instance as described in WO 97/22720.

An example of a strong, but non-covalent binding technique involves the attachment of a biotinylated Detection Sequence onto a carrier coated with streptavidin.

In order to create small, distinct, adressable areas of each of the Detection Sequences on the array, masking techniques or known microdispensing techniques may be used, for instance as described in WO 97/46313 and WO 97/22720.

Also, the Detection Sequences may be synthesized in situ on the array using solid phase nucleic acid synthesis techniques, again as described in the above reference.

After attachment of the Detection Sequences to the carrier, the array will generally be ready for use.

In step a) of the invention, the one or more Detection Sequences (or the array of the Detection Sequences) are contacted with the sample (i.e. mixture of restriction fragments) to be analysed under hybridizing conditions known per se. Suitable hybridisation conditions (i.e. buffers used, salt strength, temperature, duration) can be selected by the skilled person, on the basis of experience or optionally after some preliminary experiments. These conditions may vary, depending on factors such the size of the Detection Sequences, the CG-content of the Detection Sequences and whether the Detection Sequence or Target Sequence is bound to an array as described below.

Suitable hybridisation conditions are for instance described in Sambrook et al., Molecular Cloning: A Laboratory manual, (1989) 2nd. Ed. Cold Spring Harbour, N.Y.; Berger and Kimmel, "Guide to Molecular Cloning Techniques", Methods in Enzymology", (1987), Volume 152, Academic Press Inc., San Diego, CA; Young and Davis (1983) Proc. Natl. Acad Sci.(USA) 80: 1194; Laboratory Techniques in Biochemistry and Molecular Biology, Vol.24, Hybridization with Nucleic Acid Probes, P. Thijssen, ed., Elsevier, N.Y. (1993), as well as WO 97/43450. EP-A-0 799 897, WO 97/27317, WO 92/10092, WO 95/1195, WO 97/22720 and US-A-5,424,186 and generally may comprise temperatures between 25-70°C, preferably 35-65°C, a duration of between 1 minute and 30 hours, preferably about 15 minutes to 2 hours, and the use a suitable buffer, such as a Tris-buffer.

The hybridisation conditions are preferably chosen such that each Detection Sequence will only form a hybrid (duplex) with a Target Sequence with which the Detection Sequence is essentially complementary as defined above, if such a Target Sequence is present, and otherwise will not form any hybrid.

Especially preferred hybridisation conditions are those known per se for primer extension, such as the primer extension conditions used in minisequencing techniques, i.e. as described in WO 90/09455, WO 91/02087, WO 91/13075, WO 92/15712 and EP 0 123 513. This has the advantage that both step a) and step b) above can be carried out under the same "primer extension conditions", i.e. in a single reaction, using the same buffer, etc, optionally under repeated temperature cycling.

When the mixture of restriction fragments is contacted with a Detection Sequence, and a Target Sequence for said Detection Sequence is present, a hybrid between the Target Sequence and the Detection Sequence will be formed. Said hybrid should be such - i.e. the Detection Sequence should be designed to be complementary to the Target Sequence such - that there is at least one unpaired nucleotide of the Target Sequence directly adjacent to the 3' end of the Detection Sequence.

During step b) of the method of the invention, the at least one position corresponding to said unpaired nucleotide(s) in the Target Sequence is "filled in" with at least one nucleotide, by means of elongation of the Detection Sequence, in which the Target Sequence serves as a template for an extension reaction. Therefore, if the Target Sequence that corresponds to the specific Detection Sequence is not present, no hybrid will be formed, and said Detection Sequence will not be extended, showing that the Target Sequence was not present in the starting mixture.

In step b), the Detection Sequence is preferably extended with at most five, preferably at least most three, and most preferably only one nucleotide.

Preferably, the nucleotide used in step b) to extend the Detection Sequence (or at least one of the nucleotides used therefor) is a nucleotide or nucleotide analog that can be detected in a manner known per se, for instance by means of a detectable label, and such a nucleotide is referred to as a "Detectable Nucleotide". It is to be understood that the term "Detectable Nucleotide" also incorporates the extension of the Detection Sequence with several nucleotides, at least one of which is labeled or otherwise can be detected in a manner known per se.

Suitable labels for use in the Detectable Nucleotide are for instance described in WO 97/27317, WO 97/22720, WO 97/43450, EP 0 799 897, WO 97/31256, WO 97/27317 and WO 98/08083 and include fluorescent labels, phosphorescent labels, chemoluminescent labels, bioluminescent labels, chemical labels, biochemical labels such as enzymes, biological labels such as biotin/streptavidin, radioisotopes, spin or resonance labels, metal colloids such as gold, magnetic beads, chromogens, dyes, and similar labels.

In particular, labelling techniques and/or labeled nucleotides known per se for use in minisequencing can be used, such as those mentioned in WO 90/09455, WO 91/02087, WO 91/13075, WO 92/15712 and/or EP 0 123 513.

Furthermore, so-called "indirect" labels may be used, which are joined to the Target Sequence/Detection Sequence after hybridisation, again as for instance described in WO 97/27317.

Besides being provided with a detectable label, the Detectable Nucleotide is preferably such that - optionally in conjunction with the further components of the reaction mixture and/or the conditions used for extension of the Detection Sequence - the extension of the Detection Sequence terminates after the Detectable Nucleotide has been added to the Detection Sequence. For this purpose, chain terminating nucleotides and/or chain terminating conditions may be used, again as known per se for use in minisequencing, for instance from WO 90/09455, WO 91/02087, WO 91/13075, WO 92/15712 and/or EP 0 123 513. Also, a combination of a Detectable Nucleotide and a terminating nucleotide may be used, provided that the Detectable Nucleotide is build into the Detection Sequence before extension of the Detection Sequence is terminated.

As non-limiting examples of terminating nucleotides, deoxyribonucleoside triphosphates (dNTPs), dideoxyribonucleoside triphosphates (ddNTPs) or thionucleotides, all provided with suitable labels as described above, may be used. These include dATP, dCTP, dGTP, dUTP, dITP, ddATP, ddCTP, ddGTP, ddTTP.

The conditions for extension of the Detection Sequence with the Detectable Nucleotide may include all conditions known per se for the extension of a oligonucleotide or primer hybridized to a nucleic acid template, for instance as described in the art for minisequencing, such as in WO 90/09455, WO 91/02087, WO 91/13075, WO 92/15712 and/or EP 0 123 513. These include use of a polymerase such as E. coli DNA polymerase, Klenow fragment, bacteriophage T7 DNA polymerase, bacteriophage T4 DNA polymerase, Taq DNA polymerase and AMV transcriptase, in a suitable buffer, such as an aqueous buffer containing Mg-salts, at a temperature of 20-80°C, preferably 30-70 °C.

According to one embodiment of the invention, the mixture used in step b) for extending the Detection Sequence contains only one (type of) Detectable Nucleotide, i.e. one or more Detectable Nucleotides that are complementary to only one of A, T, C or G. Under these conditions, only those target sequences that: 1) can hybridize succesfully with the Detection Sequence; and 2) have in their sequence, on a position directly adjacent to the part of the sequence that hybridizes with the Detection Sequence, a nucleotide complementary to the Detectable Nucleotide, will lead to extension of the Detection Sequence with the Detectable Nucleotide and thereby to a positive signal, indicative for the presence of said Target Sequence in the mixture to be analysed.

In this way, when the unpaired nucleotide of the Target Sequence directly adjacent to the 3' end of the Detection Sequence in the hybrid is known, the use of a Dectectable Nucleotide corresponding to said unpaired nucleotide provides a further selection or confirmation of the identity of the Target Sequence. To improve selectivity and/or sensitivity, two or more known nucleotides adjacent to the 3' end of the Detection Sequence may be "filled in" with labeled nucleotides (preferably labeled in a distinguisable manner), i.e. in one cycle of steps a) and b) or several cycles.

According to another embodiment of the invention, the mixture used in step b) for extending the Detection Sequence but may also contain two, three or four different Detectable Nucleotides. By "different Detectable Nucleotide" is meant that each Detectable Nucleotide is complementary to a different unpaired nucleotide A, T, G or C on the Target Sequence, and each Detectable Nucleotide is labeled in such a way that it can be distinguished - i.e. using a suitable detection technique - from the other Detectable Nucleotide(s) present in the extension reaction and/or the extended Detection Sequence, so that extension of the Detection Sequence with a specific Detectable Nucleotide can be indicative for the presence of a specific nucleotide on the corresponding position of the Target Sequence.

Optionally, after the hybridization of step a), and/or after the extension reaction of step b), any restriction fragments not hybridized to a Detection Sequence, as well as any other unwanted sequences, compounds, or excess reagents, may be removed, for instance by washing the array.

After the Detection Sequence in the Target Sequence/Detection Sequence duplexes have been extended with the Detectable Nucleotide ("DN"), the resulting mixture is analysed to determine which Detection Sequence have been extended with a DN, using a suitable detection technique. This analysis may be carried out while the Detection Sequence(s) are still hybridized to the corresponding Target Sequence(s), or Target Sequence(s) may be removed/separated from the Detection Sequence(s) in a separate step prior to dectection and analysis.

When the Detection Sequences are in the form of an array, the array is analysed to determine to which areas on the array (i.e. which Detection Sequence(s)) show the Detectable Nucleotide. These areas will generally be detected as a positive signal indicating the presence of the pertinent marker in the sample.

The analysis of the array may be carried out in any manner known per se, including optical techniques, spectroscopy, chemical techniques, biochemical techniques, photochemical techniques, electrical techniques, light scattering techniques, colorimetric techniques, radiography techniques, etc., depending on the label in the Detectable Nucleotide. Suitable techniques are for instance described in WO 97/27317, WO 97/22720, WO 97/43450, EP 0 799 897, WO 97/31256, WO 97/27317 and WO 98/08083. For instance, the array may be inspected visually or by (confocal) microscopy; by spectroscopy; using photographic film, electronic detectors or a CCD camera; by colorimetric or (bio)chemical assay; or by any other suitable method, for which again reference is made to WO 97/27317, WO 97/22720, WO 97/43450, EP 0 799 897, WO 97/31256, WO 97/27317 and WO 98/08083. Automated scanning equipment based upon such techniques may also be used.

Optionally, the relative intensity or absolute magnitude of a signal generated by a Detectable Nucleotide on a specific site on the array may be used as a relative indication or an absolute measure of the amount of the corresponding Target Sequence fragment present in the original sample, for instance as described in WO 98/08083.

The analysis of the array may as such provide useful results, i.e. show the presence or absence of a genetic marker or genetic trait of interest, identify an individual, or otherwise provide information on the individual analysed, such as to which strain, variety, cultivar or race it belongs. It may also directly indicate the presence or absence of a disease state.

Optionally, the data obtained from "reading" the array may also be processed further, i.e. by comparing it to references, to earlier results or to a database, optionally using computer algorithms.

Advantageously, the method and array of the invention can be used to replace conventional fingerprinting/autoradiography analysis in AFLP. This aspect of the invention comprises steps (a) - (e) of the general AFLP-method described above, in which step (e) is carried out by the extension/elongation reaction of steps a) to c) above.

Compared to conventional fingerprinting/autoradiography, using the method and array of the invention may be faster, and several markers that would require generating several separate fingerprints could be detected on a single array. All this makes the method and arrays of the invention especially suited for routine and/or high throughput screening, for instance in plant breeding.

Also, the array of the invention can conveniently be provided as a kit of parts comprising the array and other components for use with the array, such as hybridization buffers, extension buffers, polymerase, labeled nucleotides, containers/packaging and manuals, as well as components for AFLP-kits known per se. The array of the invention may even be in the form of a hand-held device such as a dipstick.

The array of the invention can be used to analyse any kind of nucleic acid sequence or mixture of nucleic acid sequences, including but not limited to plant-derived sequences, animal-derived sequences, human-derived sequences, microbial sequences, yeast sequences, sequences from fungi and algi, and viral sequences, including genomic DNA, cDNA, structural genes, regulatory sequences and/or parts thereof. Prior to use in the method of the invention, these starting nucleic acids are restricted, most preferably using the same restriction enzymes as used in generating the sequences from which the Detection Sequence(s) were derived, thereby providing a mixture of restriction fragments that can be used directly in step a) above.

Also, in a highly preferred embodiment of the invention, in analysing a mixture of restriction fragments suspected of containing at least one Target Sequence, the restricted mixture is amplified prior to contacting with the Detection Sequence(s) in step a) above. Preferably, said amplification is by "AFLP", by which in this context is more generally meant that the starting DNA is cut using at least one restriction enzyme and then amplified using adapters and primers. This leads to a reduction of sample complexity, giving less background noise. Said (AFLP) amplification may or may not involve the use of selective AFLP primers. High-throughput analysis of AFLP markers can be achieved particularly when Target Sequences are generated using less selective nucleosides then used for detection of the same AFLP markers by PAGE.

In principle, method and arrays of the invention can be applied to, and can be used for, any purpose for which a polymorphic marker can be used and/or identified. This includes, but is not limited to, all the uses described in the art for polymorphic markers in known DNA-fingerprinting, genotyping, profiling and DNA-identification techniques. The method and arrays of the invention are of course especially suited for applications for which an AFLP-marker can be used and/or identified, including those mentioned above and in EP-A-0 534 858 and the co-pending European applications EP 0 976 835 and EP 0 974 672.

Possible fields of use are for instance plant and animal breeding, variety or cultivar identification, diagnostic medicine, disease diagnosis in plants and animals, identification of genetically inherited diseases in humans, family relationship analysis, forensic science, organ-transplant, microbial and viral typing such as multiplex testing for strains of infectious diseases; as well as the study of genetic inheritance, gene expression, mutations, oncogenes and/or drug resistance; or for mRNA detection.

As already mentioned above, in these applications, it is envisaged that arrays of the invention can be developed that carry Detection Sequences representative for most or even all markers of interest for a specific genotyping collection, such as for a specific species. Other arrays of the invention may contain Detection Sequences that are representative for most or all markers necessary to classify an individual within a genotyping collection, i.e. as belonging to a certain species, subspecies, variety, cultivar, race, strain or line, or to study the inheritance of a genetic trait or property. Also, an array of the invention may contain Detection Sequences representative of markers that are indicative of the presence, the absence or the state of a genetically determined or genetically influenced disease or disorder, including cancer, oncogenes and oncogenic mutations. Such an array may then be used for diagnostic purposes.

In a further aspect, the invention relates to results and/or data obtainable by analysing a mixture of restriction fragments with the method of the invention. These results or data may for instance be in the form of an image, of a score, of digital or analog data, or in another suitable form, and may optionally be stored on a suitable data carrier, including paper, photographic film, computer disc of files, a database, etc.. This data may be as directly obtained from analysing or scoring the array, or may have been processed further.

The invention will now be further illustrated by means of the following non-limiting Experimental Part, as well as by the enclosed Figures, which show:

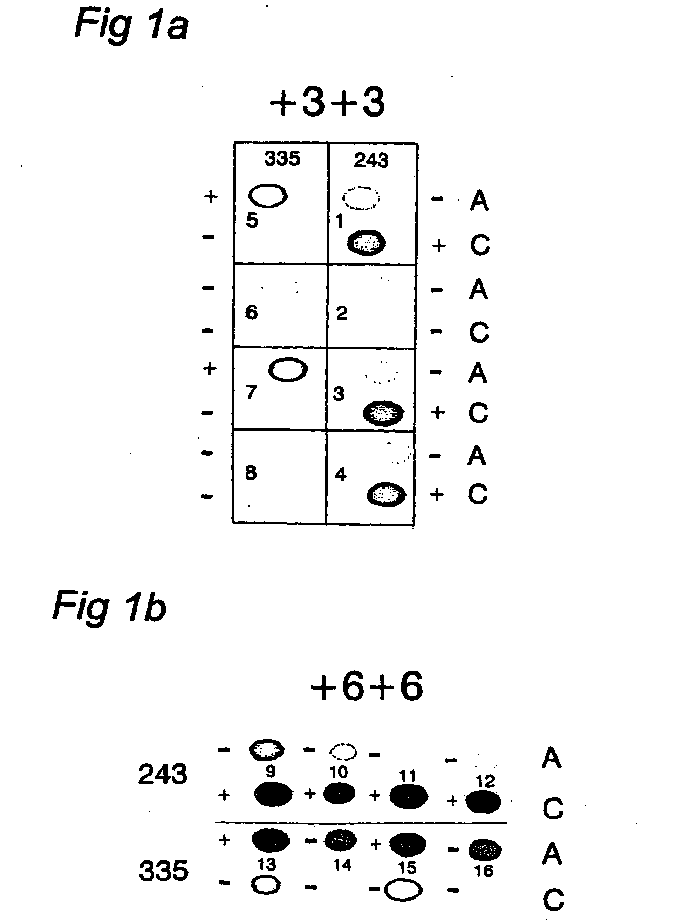

- Fig. 1:: Detection of 2 polymorphic fragments 243 and 335 in +3+3 (fig. 1A) and +6+6 (fig. 1B) AFLP template (see example 3). +3+3 implies that an AFLP +3+3 reaction was used as template for the primer extension reaction. +6+6 implies that an AFLP +6+6 reaction was used as template for the primer extension reaction. A implies that dATP was offered in the primer extension reaction; C implies that dCTP was offered in the primer extension reaction. 1 to 16 refer to the numbers of the samples. + implies that primer extension occurred, - implies that primer extension did not occur.

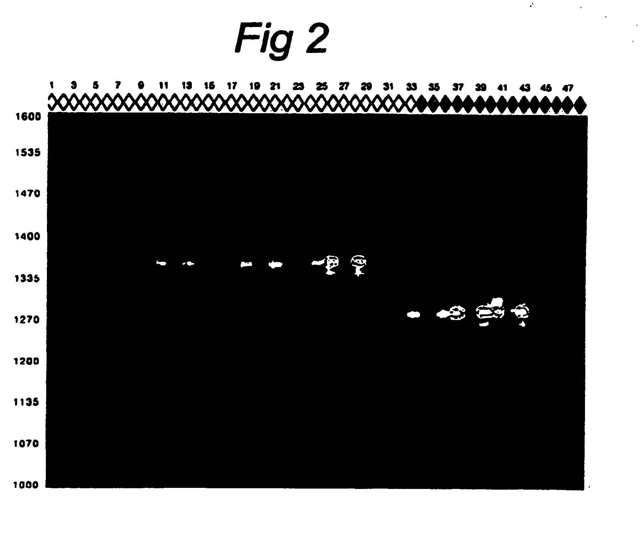

- Fig.2:: Detection of 2 polymorphic fragments 243 and 335 using fluorescently labeled ddNTPs (FAM ddCTP and JOE ddATP) in templates of decreasing complexity for fragment 243 generated with +3+3, +4+3, +3+4, +4+4, +6+4, +6+5, +6+6 primer combinations and for fragment 335 with +3+3, +3+4, +6+4, +6+6 primer combinations. Images were produced on an ABI Prism 377 DNA sequencer. Primer extension reactions were performed on three samples: two samples having the fragment and one sample not having the fragment in the order +, -, +.

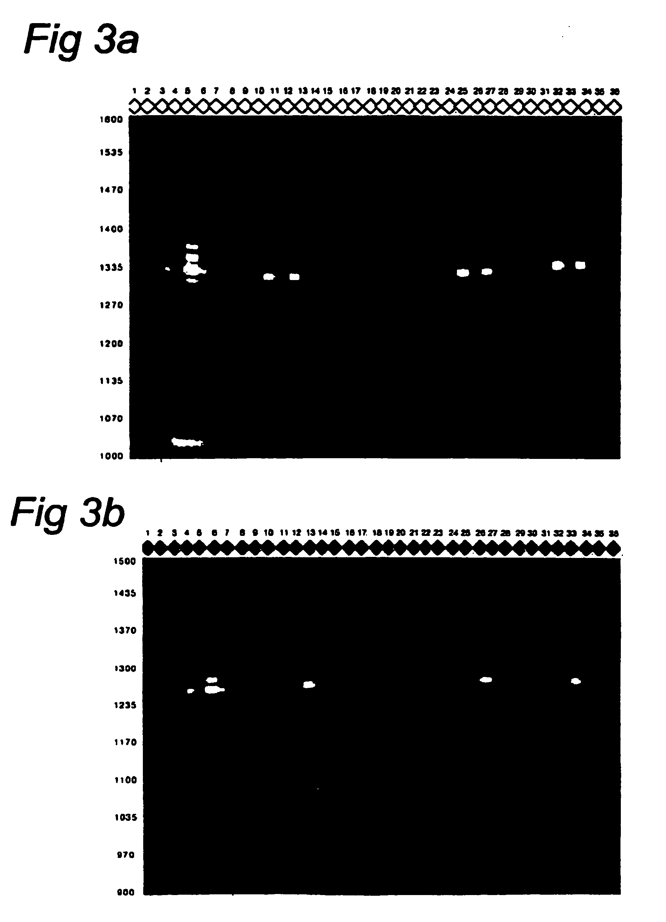

- Fig.3:: Detection of polymorphic AFLP fragments 243 (Fig. 3A) and 335 (Fig. 3B) using fluorescently labeled ddNTPs (FAM ddCTP and JOE ddATP) and detection step primers in which some nucleosides have been replaced by inosine residues (I). Templates were of decreasing complexity, generated with respectively +3+3, +3+4, +6+4, +6+6 primer combinations. Primer extension reactions were performed on two samples: one samples having the fragment and one sample not having the fragment in the order +, - .

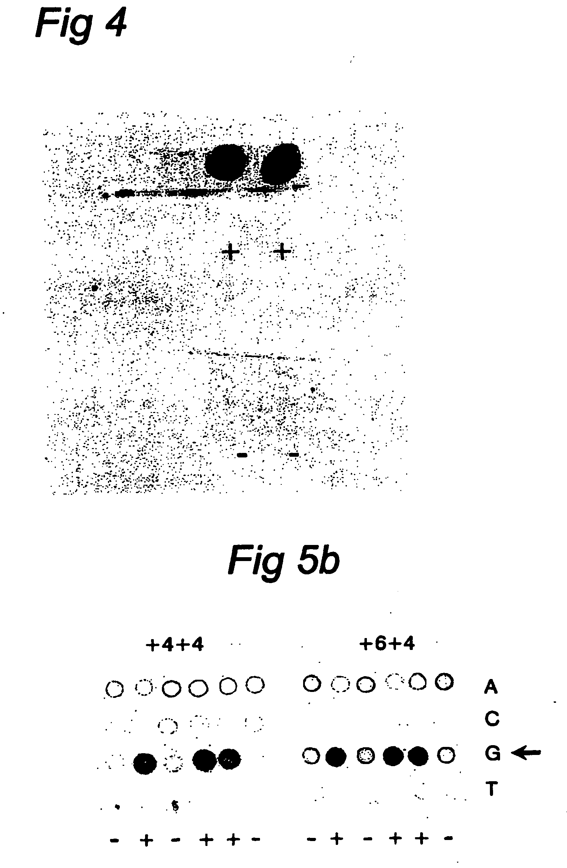

- Fig.4:: Detection of polymorphic AFLP fragment 243 generated with +3+3 AFLP primer combinations EcoAAC/ MseCAA in ds AFLP template. Detection step primers were attached to glass strips. The primer extension step was performed by temperature cycling in a PCR tube. + implies that primer extension occurred, - implies that primer extension did not occur. (see example 6)

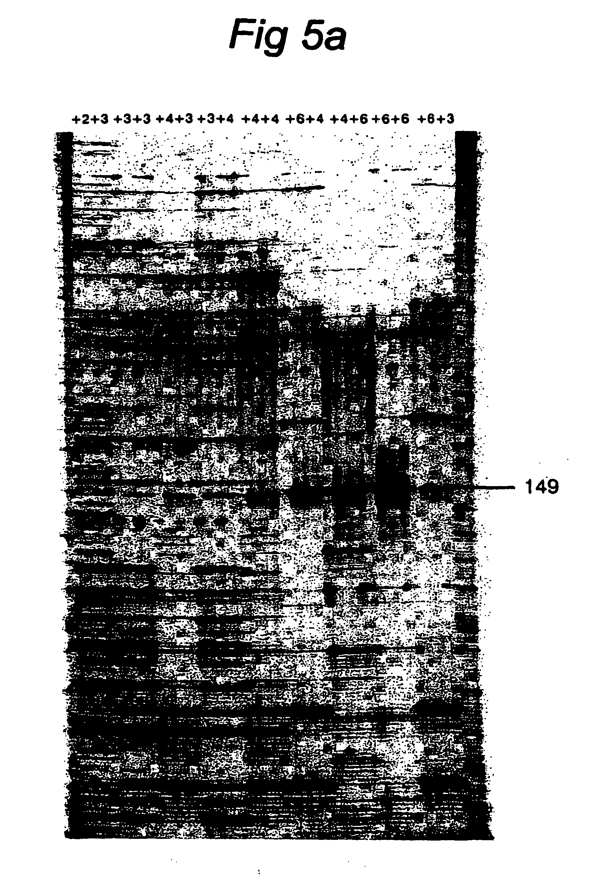

- Fig.5A:: AFLP fingerprint generated with primer combinations with increasing numbers of selective nucleosides, (respectively +2+3, +3+3, +4+3, +3+4, +4+4, +6+4, +4+6, +6+6, +6+3) for panels of 4 samples: one sample not having the fragment and three samples having the fragment in the order (-, +, +, +). Primer extensions were chosen to generate polymorphic band 149 as indicated with an arrow.
- Fig. 5B:: Detection of polymorphic fragment 149 from fig. 5A on template generated with +4+4 and +6+4 primer combinations. Primer extension assays were performed on 6 rice individuals with three sample not having the fragment and three samples having the fragment in the order (-, +,-, +, +, -) with the 4 ddNTPs (A,C,G,T) as indicated. A positive reaction only occurred if the primer was extended with ddGTP.

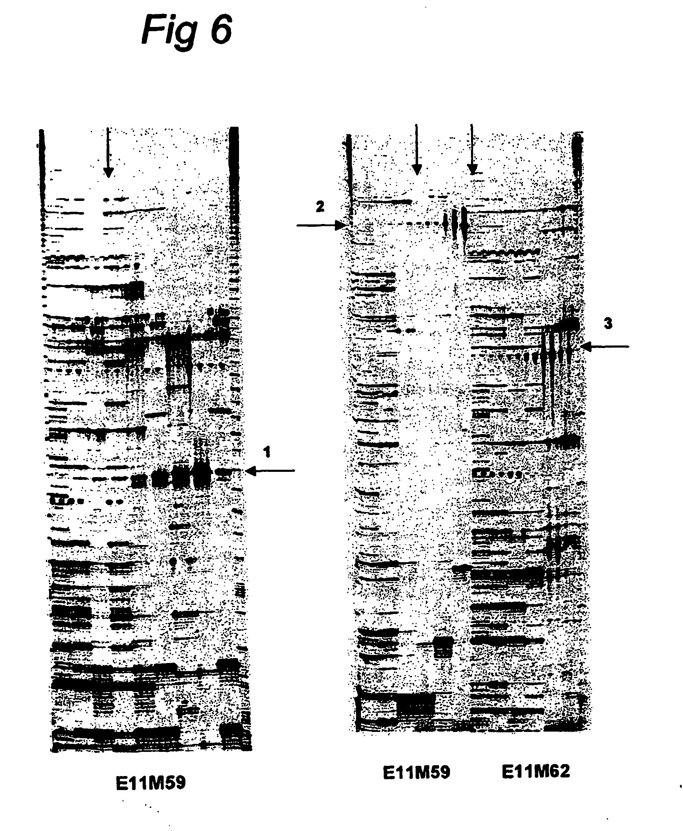

- Fig.6:: AFLP fingerprint generated with primer combinations with increasing numbers of selective nucleosides, for panels of 4 individuals and starting with +2+3 amplified template. Primer combinations were chosen to generate the polymorphic bands (Marker 1 and 2 and 3) as indicated. Arrows on top indicate maximal complexity of the AFLP reaction from which the indicated marker could still be detected by minisequencing (detection limit).

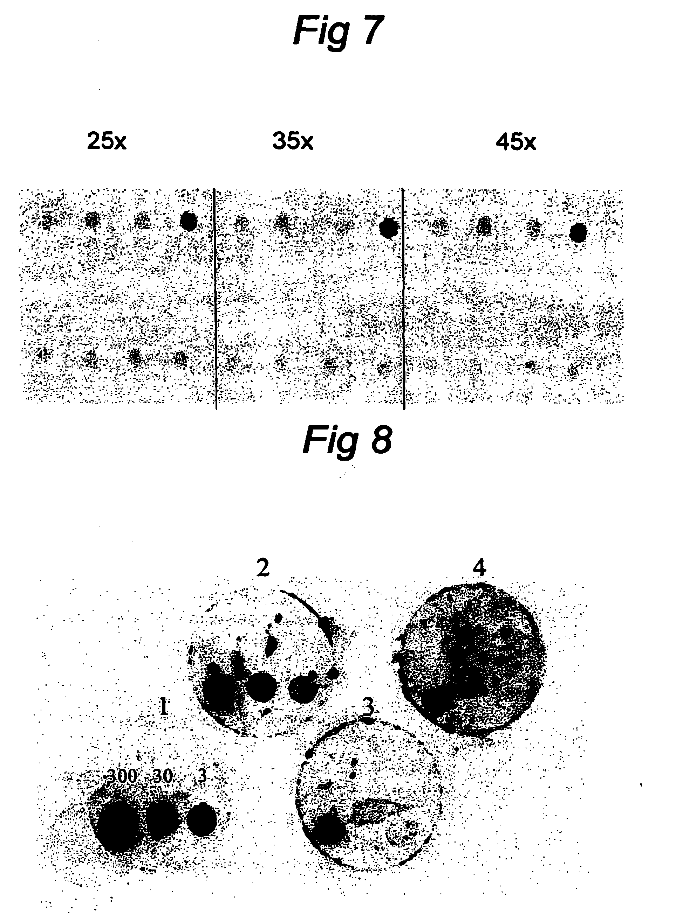

- Fig. 7:: Minisequencing assays on ds DNA with an increasing number of PCR cycles on 3 negative individuals (not having the target fragment) and one positive individual (having the target fragment). The reaction on individual 4 shows that signal increases when the number of PCR cycles increases.
- Fig. 8:: Minisequencing on glass slides: in every well primers were spotted in increasing concentrations of 300, 30 and 3 pmol, respectively. Minisequencing occurred with increasing concentrations of 0.05, 0.5, 5 and 50 pmoles of ss template in wells 1, 2, 3 and 4, respectively.

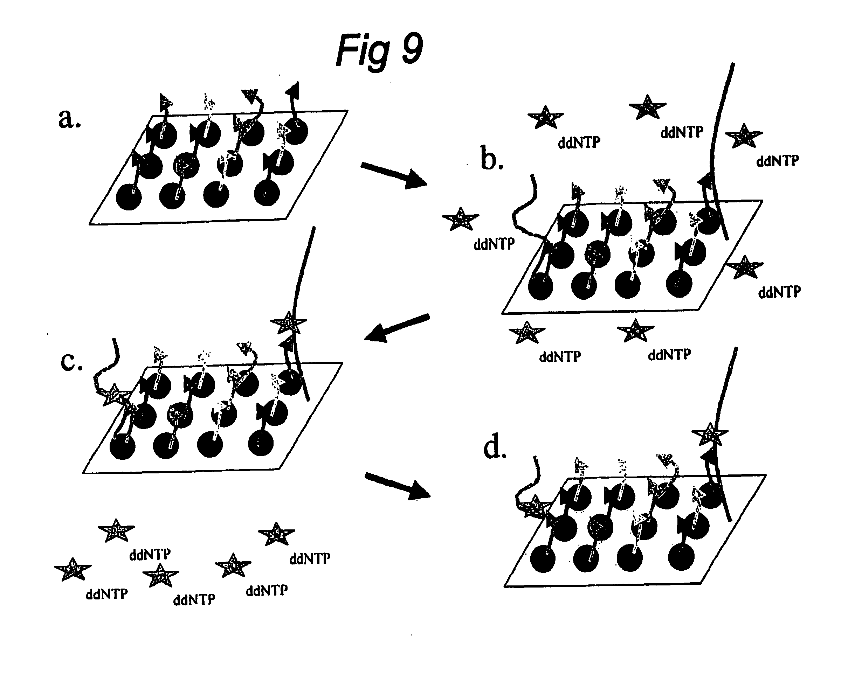

- Fig. 9:: Schematic overview of the method of the invention. Fig. 9a depicts the binding of an oligonucleotide to a chip for every AFLP marker to be detected. Next, the hybridization of an AFLP reaction mixture is shown in Fig. 9b. Fig. 9c depicts the performance of a minisequencing reaction and the removal of excess label by washing resulting into a chip ready for scanning and detection of AFLP markers, as illustrated in Fig. 9d.

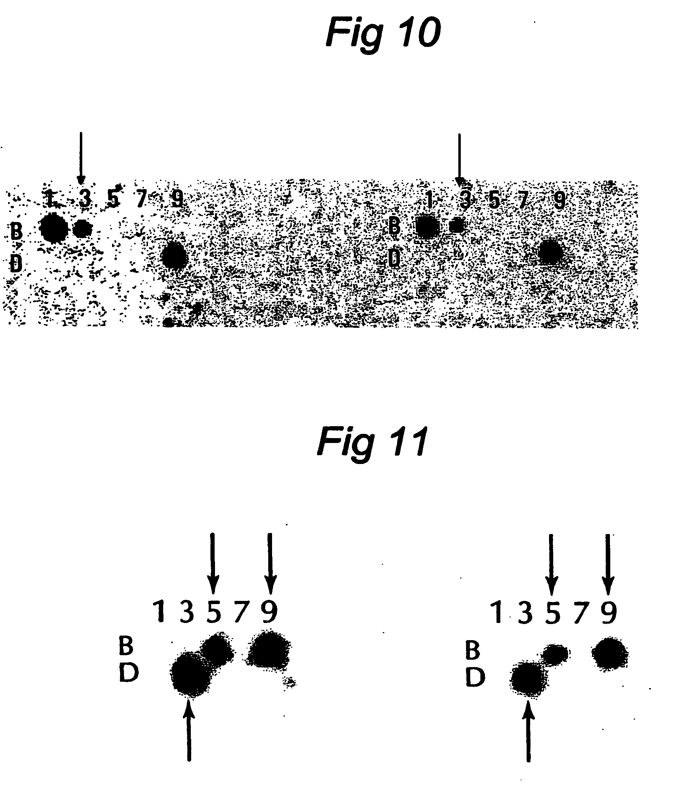

- Fig. 10:: Image of the minisequencing reaction of Example 8, scanned for 180 sec at the FITC and Cy-3 channel using a Genetac 1000 microarray slide scanner.
- Fig. 11:: Image of the minisequencing reaction of Example 9. Patterns were visualized using an Fuji BAS-2000 phosho image analysis system.

### EXPERIMENTAL PART

### Example 1: General protocols

AFLP templates were prepared according to standard procedures (Vos et al. 1995 Nucleic Acids Research vol 23, no. 21: 4407-4414; Zabeau and Vos; European Patent Application, EP 0534858), using *EcoR*I and *Mse*I as the enzyme combination. AFLP reactions were preceeded by preamplifications with one selective nucleoside on each primer. AFLP reactions were performed with the number of selective nucleosides on each primer as indicated e.g. +3+3 implies 3 selective nucleosides on each primer, the first number relates to the *EcoR*I primer, the second number relates to the *Mse*I primer.

For sequence analysis, AFLP-fragments were cut out from a dried polyacrylamide gel, reamplified with one primer having M13 reverse primer sequence extended with the sequence of the constant part of the AFLP EcoRJ primer and an Msel AFLP primer without selective nucleosides and reamplified fragments were sequenced directly from the M13 reverse primer. Sequencing reactions were performed using the dye-terminator sequencing kit purchased from Perkin-Elmer and were analyzed on an ABI 377 sequencer. Subsequently, detection step primers were designed. Sequence analysis also revealed the nature of the dNTP or ddNTP to be added for primer extension.

When AFLP reactions were used as template for primer extension reactions, AFLP reactions were performed without radioactively or fluorescently labeled primers and with 75ng of both primers in a volume of 50 ul.

The primer extension reaction on ss DNA was performed according to Syvänen et al. (Syvänen et al. 1990 Genomics 8: 684-692; Syvänen et al. 1993 Am.J.Hum.Genet.52: 46-59) with the following modifications. 10 to 25 ul of AFLP reaction mix was used for an primer extension reaction. AFLP reactions were performed using one biotinylated primer ("bio-primer") and one standard primer and biotinylated AFLP products were collected on streptavidine-coated magnetic beads (Dyna beads, Dynal, Norway) or in streptavidin-coated microtiter plate wells (Labsystems, Helsinki, Finland). Subsequently, the primer extension reaction was performed on either 1) the biotinylated strand ("bio-strand") that remained bound to the beads/ microtiterplate wells after denaturation with NaOH or 2) the non-bio-strand that could be seperated from the bio-strand by boiling and pipetting off the supernatant. This was performed after purification of the AFLP fragments by adding shrimp alkaline phosphatase and exonucleaseI to get rid of any leftover primer and dNTPs; to 50 ul AFLP reaction 50 ul of the following mix was added: 4 u shrimp alkaline phophatase (Amersham Life Science, Cleveland, Ohio), 2 u exonucleaseI (Amersham, Pharmacia, Biotech) I, 5 ul shrimp alkaline phosphate buffer, and water to 50 ul. This was incubated for 30 minutes at 37 oC, subsequently enzymes were inactivated at 95 °C. (Chen et al. 1997 Proc. Natl. Acad. Sci. USA, vol 94, 10756-10761).

The primer extension reaction on the bio-strand was done in 10 mM Tris-HCl pH 9.5, 5 mM KCI, 2 mM MgCl₂, 0.002% Tween-20, 2 µl of 5 µM detection primer, 0.3 µl labeled ³³P ddNTP (Amersham), 3.2 units of Thermosequenase and water to a total volume of 50 µl. The reaction was incubated for 10' at 50 °C, wells/beads were washed and minisequencing products were released from the beads by denaturation in 20 ul formamide dye (80% formamide with 10mg/ml Blue dextran) at 94 °C, spotted on Whatman paper and exposed to Fuji phosphoimage screens for 16 hours. Patterns were visualized using a Fuji BAS-2000 phosphoimage analysis system (Fuji Photo Film Company Ltd, Japan).

The minisequencing reaction on the non-biostrand was performed in the same way as described above, however primer extension reactions were performed on glass slides with modified primers attached to them (vide the non-limiting schematic drawing of Figure 9). Non- biostrands of AFLP fragments were added to the reaction mixture. Coating of glass slides and binding of primers was performed as described by Guo et al. (Guo et al. 1994 Nucleic Acids Research vol 22, no. 24: 5456-5465.). 1 µl of primers was spotted manually. Detection step primers were 5'amino and 5' (T)₁₅ modified. Slides were washed and exposed to Fuji phosphoimage screens for 16 hours. Patterns were visualized using a Fuji BAS-2000 phosphoimage analysis system (Fuji Photo Film Company Ltd, Japan).

The primer extension reaction on ds DNA was performed according to Syvänen *et al.* (1990, 1993) with the following adaptations. AFLP reactions were performed and treated with shrimp alkaline phosphatase and exonucleaseI to get rid of any leftover primer and dNTPs (Chen et al. 1997). Subsequently, the primer extension reaction was performed in 50 ul in 10 mM Tris-HCl pH 9.5, 5 mM KCI, 2 mM MgCl₂, 0.002% Tween-20, 2 µl of 5 µM modified detection primer, 0.3 µl labeled ³³P ddNTP (Amersham), 2.5 µl 2µM ddNTPs, 3.2 unit of Thermosequenase. The PCR profile consisted of 35 cycles 15" 95 °C , 30 " 58 °C (Chen et al. 1997). Primer extension reactions were performed on glass strips with primers attached to them in PCR tubes. Coating of glass strips and binding of primers was performed as described by Guo *et al.* (1994). 1 µl of primers was spotted manually. Detection step primers were 5'amino and 5' (T)₁₅ modified.

Glass strips were washed and exposed to Fuji phosphoimage screens for 16 hours. Patterns were visualized using a Fuji BAS-2000 phosphoimage analysis system (Fuji Photo Film Company Ltd, Japan).

### Example 2: Oligo synthesis

Oligonucleotides were synthesized according to standard procedures, or they were purchased from MWG - Biotech GmbH (Germany).

### Example 3: Detection of two polymorphic AFLP fragments in tomato.

This example illustrates the detection of two polymorphic AFLP fragments in tomato, 243 and 335, generated with +3+3 AFLP primer combinations bio-EcoAAC/MseCAA and bio-EcoACT/ MseCAC respectively, or +6+6 AFLP primer combinations bio-EcoAACCAC/ MseCAACAG and bio-EcoACTTTT/ MseCACGAA, respectively.

AFLP reactions were performed using a biotinylated Eco -primer and one standard Mse-primer and biotinylated AFLP products were captured in streptavidin-coated microtiter plate wells. Subsequently, the primer extension reaction was performed on the bio-strand as described above, however using ³²P labeled dNTP (0.1ul/reaction; Amersham). For polymorphic fragment 243 in +3+3 AFLP template 3 samples having the fragment (1,3,4; Fig 1A) and 1 sample not having the fragment (2) were assayed; for polymorphic fragment 335 2 samples having the fragment (5,7) and 2 samples not having the fragment (6,8) were assayed. For polymorphic fragment 243 in +6+6 AFLP template 4 samples having the fragment were assayed (9,10,11,12; Fig.1B); for polymorphic fragment 335 2 samples having the fragment (13,15) and 2 samples not having the fragment (14,16) were assayed. For fragment 243 detection step primer 1 was used; which has to be extended with an C, for fragment 335 detection step primer 2 was used, which has to be extended with an A. Reactions were performed with both dATP and dCTP. Extension products were spotted on Whatmann 3MM paper. Results show that a positive reaction only occurs if the targeted AFLP fragment is present and if primers according to the sequence information are extended with the appropriate nucleoside triphosphate.

The sequence of the constant region for the *Eco*-primers was (5'-3'):
GACTGCGTACCAATTC

The sequence of the constant region for the *Mse*-primers was (5'-3'):
GATGAGTCCTGAGTAA
AFLP primers to generate fragment 243 (5'-3'):
   Bio-Eco+3: EcoAAC
   Mse+3: MseCAA
   BioEco+6: EcoAACCAC
   Mse+6: MseCAACAG
   Detection step primer 1 to detect fragment 243 if extended with dCTP (5'-3'):
   AGCAGTAGCAACCACTTCAGCC
AFLP primers to generate fragment 335 (5'-3'):
   Bio-Eco+3: EcoACT
   Mse+3: MseCAC
   Bio-Eco+6: EcoACTTTT
   Mse+6: MseCACGAA
   Detection step primer 2 to detect fragment 335 if extended with dATP:
   ATCCGGCCAGTTATACC

### Example 4: Detection of polymorphic AFLP fragment 243 and 335 using fluorescently labeled ddNTPs.

AFLP reactions were performed with primer combinations with increasing numbers of selective nucleosides, for panels of 3 individuals (+, -, +). AFLP primer extensions were chosen to generate fragment 243 and 335, respectively. AFLP reactions were performed using a biotinylated EcoRI-primer and one standard MseI-primer and biotinylated AFLP products were captured on streptavidine-coated magnetic beads. Subsequently, the primer extension reaction was performed on the bio-strand as described above, however using fluorescently labeled ddNTPs (0.1ul/ reaction Joe-ddATP, Joe-ddCTP, NEN, Boston, USA). Detection step primer and extensions were as in example 3. Extension products were analyzed on an ABI sequencing gel. Results show that a positive reaction only occurs if the targeted AFLP fragment is present. (Fig.2)
AFLP primers to generate fragment 243 (5'-3'):
   Bio-Eco+3: EcoAAC
   BioEco+4: EcoAACC
   BioEco+6: EcoAACCAC
   Mse+3: MseCAA
   Mse+4: MseCAAC
   Mse+5: MseCAACA
   Mse+6: MseCAACAG
   Detection step primer 1 to detect fragment 243 if extended with ddCTP (5'-3'):
      AGCAGTAGCAACCACTTCAGCC
AFLP primers to generate fragment 335 (5'-3'):
   Bio-Eco+3: EcoACT
   Bio-Eco+6: EcoACTTTT
   Mse+3: MseCAC
   Mse+4: MseCACG
   Mse+6 : MseCACGAA
   Detection step primer 2 to detect fragment 335 if extended with ddATP (5'-3'):
      ATCCGGCCAGTTATACC

### Example 5: Detection of polymorphic AFLP fragments 243 and 335 using fluorescently labeled ddNTPs and detection step primers in which some nucleosides have been repaced by inosine residues.

AFLP reactions were performed with primer combinations with increasing numbers of selective nucleosides, for panels of 2 individuals (+, -). Primer extensions were chosen to generate fragment 243 and 335, respectively. AFLP reactions were performed using a biotinylated Eco -primer and one standard Mse-primer and biotinylated AFLP products were captured on streptavidine-coated magnetic beads (Dynal). Subsequently, the primer extension reaction was performed on the bio-strand as described in example 2, however using both standard AFLP primers and degenerate AFLP primers in which some selective nucleosides had been replaced by inosine, as detection step primers. For fragment 243 detection step primers had to be extended with an C, for fragment 335 detection step had to be extended with an A. Fluorescently labeled ddNTPs were used (0.1ul/ reaction (Joe ddATP, Fam ddCTP, NEN, Boston, USA). Extension products were analyzed on an ABI sequencing gel. Results show that a positive reaction only occurs if the targeted AFLP fragment is present and that standard AFLP primers can be replaced by primers in which nucleosides have been replaced by 1, 2, or 3 inosine residues, respectively (Fig.3A for fragment 243; Fig. 3B for fragment. 335). Non-selective nucleosides are only introduced in primers at positions that have been fixed in the AFLP reaction used as template for the primer extension reaction.
AFLP primers to generate fragment 243 (5'-3'):
   Bio-Eco+3: EcoAAC
   BioEco+6: EcoAACCAC
   Mse+3: MseCAA
   Mse+4: MseCAAC
   Mse+6: MseCAACAG
   Detection step primers to detect fragment 243 if extended with Fam ddCTP (5'-3):
   Mse+6: MseCAACAG
   Mse+6: MseIIICAG
   Mse+6: MseIIACAG
   Mse+6: MseIAACAG
AFLP primers to generate fragment 335 (5'-3'):
   Bio-Eco+3: EcoACT
   Bio-Eco+6: EcoACTTTT
   Mse+3: MseCAC
   Mse+4: MseCACG
   Mse+6: MseCACGAA
   Detection step primers to detect fragment 335 if extended with Joe-ddATP (5'-3'):
   Mse+6: MseCACGAA
   Mse+6: MseIIIGAA
   Mse+6: MseIICGAA
   Mse+6: MseIACGAA

### Example 6: Detection of polymorphic AFLP fragment 243 generated with +3+3 AFLP primer combinations EcoAAC/ MseCAA in ds AFLP template.

+3+3 AFLP reactions were performed as described in example 3, however with standard AFLP primers (no biotinylated primer was used).

Primer extension reactions were performed as described for ds DNA with temperature cycling. 2 samples having the fragment and 2 samples not having the fragment were assayed; modified detection step primer 1 was used. Results show that a positive reaction only occurs if the targeted AFLP fragment is present (Fig.4).
AFLP primers to generate fragment 243 (5'-3'):
   Eco+3: EcoAAC
   Mse+3: MseCAA
   Modified detection step primer 1 to detect fragment 243 if extended with ddCTP (5'-3): NH2-(T)15 AGCAGTAGCAACCACTTCAGCC

### Example 7: Detection of polymorphic AFLP fragment 149 in rice, using an AFLP +6 primer as detection step primer

AFLP fingerprints were generated with primer combinations with increasing numbers of selective nucleosides, for panels of 4 individuals (-, +, +, +; Fig 5A). Primer extensions were chosen to generate fragment 149 as indicated. AFLP fingerprints show the complexity of AFLP reactions dependent on the number of selective nucleosides.

AFLP reactions used as template for primer extension reactions were performed as described in example 3 using a biotinylated EcoRI -primer and one standard MseI-primer and biotinylated AFLP products were captured in streptavidin-coated microtiter plate wells (Labsystems, Helsinki, Finland. Subsequently, the primer extension reaction was performed on the bio-strand using all four 33P labeled ddNTPs and using a standard AFLP +6 primer, that had to be extended with a G. Primer Mse CTAAAT was used as detection step primer in +6+4 and +4+4 AFLP reactions on 6 individuals with three sample not having the fragment and three samples having the fragment in the order (-, +, -, +, +, -, Fig. 5B)

Results show that a positive reaction only occurs if the targeted AFLP fragment is present and if primers according to the sequence information are extended with the appropriate nucleoside triphosphate.
AFLP primers to generate fragment 149 (5'-3'):
   Eco+2: Eco-AA
   Eco+3: Eco-AAC
   (bio)-Eco+4: (bio)-Eco-AACC
   (bio)-Eco+6: (bio)-Eco-AACCTT
   Mse+3: Mse-CTA
   Mse+4: Mse-CTAA
   Mse+6: Mse-CTAAAT
   Detection step primer: Mse+6 (5'-3'): Mse-CTAAAT

### H. Detection of AFLP markers by minisequencing

Minisequencing was explored as a method to detect AFLP fragments. Minisequencing using AFLP template can be applied as 1) a method to adress sensitivity of minisequencing since the number of template fragments can easily be varied; 2) a non-random method for AFLP marker detection by using predetermined sequence information; 3) a random method for marker detection by using extended AFLP primers. So far, minisequencing as a method to detect AFLP fragments has been explored using predetermined sequence information for the design of detection primers, that were either extended AFLP primers, or internal detection primers. Often +6 primers do not detect unique fragments as was shown by determining the +6 sequences for the EcoRI-primer as well as for the MseI-primer for all fragments in 2 fingerprints generated with an EcoR1 +2 and a MseI +3 primer. The optimal number of selective nucleosides added to AFLP-primers when used as detection primers remains to be determined.

### I. Sensitivity of minisequencing detection of AFLP fragments in an AFLP template

To determine sensitivity of minisequencing on ssDNA compared to sensitivity of detection in a standard AFLP gel, AFLP reactions were carried out using increasing numbers of selective nucleosides for 3 AFLP markers (Figs 6 and 7). Minisequencing to detect the 3 AFLP markers was applied following the protocol as described for minisequencing on ss template using the bio-strand. +6 AFLP primers were used as detection primers. It was shown that the maximum complexity of the template was dependent on the marker to be detected: for marker 1 this was +6+4 amplified template; for marker 2 this was +6+4 for marker 3 this was +2+3 amplified template. This implies a detection limit at 10¹⁰ molecules, which is less sensitive than is claimed for detection on microarrays in gene expression studies (10⁶ molecules).

### J. Minisequencing on dsDNA

To be able to increase the complexity of the template and to simplify the minisequencing protocol, minisequencing was performed on ds template using a two-step PCR cycle profile: a denaturation step followed by the primer-elongation step: in this way template could be reused every cycle and the amount of signal generated by the elongated primer increased (Fig. 8). This adaptation of the protocol is a time-saving step since there is no need to make single-stranded template before the minisequencing reaction. Detection primers were biotin-labeled to easily purify minisequencing reaction products as an alternative for amino-labeled detection primers on a solid support.

### K. Solid phase detection of minisequencing products on glass slides

A protocol for the covalent binding of 5'-aminoprimers to glass was explored (Guo et al. 1994), since so far minisequencing reactions on glass were unsuccessful due to a lot of background. Primer binding was checked by radioactively labeling of primers with an internal aminogroup. It was shown that binding occurs. In a minisequencing reaction using a ss 60-mer oligo as a template, it was shown that following the protocol for ss non-biostrand minisequencing, reaction products were detected dependent on the amount of template offered and dependent on the amount of oligo attached to the slide.
Minisequencing using +3+3 amplified template and using an internal detection primer following the protocol for minisequencing on ds template was performed on glass strips in a PCR tube and it was shown that specific products were obtained.

### L. Conclusions

The minisequencing protocol was improved by using dsDNA and performing minisequencing in a number of cycles to increase sensitivity of marker detection. Minisequencing can now be performed on glass slides, which is an important step towards multiplex detection of AFLP-and other markers on chips. The current sensitivity is similar to sensitivity of marker detection in AFLP using polyacrylamide gels. It is anticipated that by using new microarray and chip technology sensitivity of detection can be improved.

### EXAMPLE 8. The development of a fluorescent AFLP marker detection system.

### 1. Description of biological materials

The biological materials used are rice lines IR20 and 6383.

### 2. EcoRI/MseI AFLP template preparation

AFLP templates were prepared using the restriction enzymes EcoRI and MseI and preamplification reactions were carried out according to standard procedures described by Vos et al., (Nucleic Acids Research 23: no 21, pp. 4407-4414, 1995; and patent application EP0534858).
Final selective amplification reactions were carried out from a 20-fold diluted +1/+1 preamplification mixture using an EcoRI selective +2 primer in combination with a MseI +3 primer.

| | AFLP marker name | Primer Combination Size (basepairs) Parent line | | |
|---|---|---|---|---|
| 1. | 1A1 | E11/M48 | 571 | IR20 |
| 2. | 1A8 | E11/M48 | 264 | IR20 |
| 3. | 1A11 | E11/M48 | 187 | IR20 |
| 4. | 1B1 | E11/M48 | 154 | IR20 |
| 5. | 1G6 | E11/M49 | 342 | 6383 |

AFLP markers 1 through 5 were excised, reamplified and cloned. The colonies were amplified and AFLP templates were made, all according to standard procedures. Preamplification reactions were carried out using an EcoRI +0 primer in combination with a biotinylated Mse +0 primer. The DNA was purified with Shrimp Alkaline Phoshatase and Exonuclease I as refered to in Example 1.
The sequences of the adapters, AFLP preamplification primers and (selective AFLP) amplification primers used are as follows:
Msel adapter:
   92A 18: 5'-GACGATGAGTCCTGAG-3'
   92A19: 3'-TACTCAGGACTCAT-5'
MseI + 0 biotinylated AFLP primer:
   93E40: *-5'-GATGAGTCCTGAGTAA-3'
MseI + I preamplification AFLP primer:
   M01 : 5'-GATGAGTCCTGAGTAAC-3'
MseI +3 selective amplification AFLP primers:
   M48: 5'-GATGAGTCCTGAGTAACAC-3'
   M49: 5'-GATGAGTCCTGAGTAACAG-3'
EcoRI adapter:
   91 M35: 5'-CTCGTAGACTGCGTACC-3'
   91M36: CATCTGACGCATGGTTAA-5'
EcoRI + 0 preamplification AFLP primer:
   E00L: 5'-GTAGACTGCGTACCAATTC-3'
EcoRI + 1 preamplification AFLP primer:
   E01: 5'-GACTGCGTACCAATTCA-3'
EcoRI +2 selective amplification AFLP primer:
   E11:5'-GACTGCGTACCAATTCAA-3'

### 3. Oligo synthesis

Oligonucleotide minisequencing detection primers were purchased from MWG- Biotech GmbH. They were 5' amino-modified and they have a poly T tail of 15 nucleotides at the 5' end.
The sequences of the oligonucleotides are as follows:
99f41 :5'-(T)₁₅TGGCTGGCAACGAGCGACA-3
99f42: 5'-(T)₁₅TTCACCCGCCGGTTAGTTTC-3
99f43: 5'-(T)₁₅ACTGTCCGCTCTCGCATTCA-3
99f44:5'-(T)₁₅GATCACGACATCACGTTGCG-3
99f45: 5'-(T)₁₅ATTGCGAGCCACATCGTTCC-3
99f46: 5'-(T)₁₅GGCCTGAAACGCTGGGTTG-3
99f47: 5'-(T)₁₅TTTTCTCGGCTTTTCTTTCT-3

### 4. The preparation of the slides

3D-Link activated slides (SurModics) were used and processed according to the manufacturer's instructions. Oligonucleotide minisequencing detection primers were printed using a genetic microsystems QMS417 microarrayer at a concentration of 0.8 µg/µl (±100 pmol/ µl) in 150 mM sodium phospate pH 8.5.

### 5. Minisequencing

Minisequencing on ss AFLP reactions was performed according to Syvänen et al. (1990, 1993) with the following modifications. The biotinylated AFLP products were collected on streptavidine-coated magnetic beads (Dynal). Subsequently, minisequencing was preformed on the non biotinylated strand that could be separated from the biotinylated stand by boiling and pipetting off the supematent. The minisequencing reaction was carried out with 20 µl ss reaction of AFLP marker 1A1 in 10 mM TRIS-HCl pH 9.5, 50 mM KCI, 20 mM MgCl₂, 0.02 % Tween-20, 2 µl 0.01 mM FAM ddATP, 4.5 units of Thermosequenase and water to a total volume of 40 µl. The reaction was incubated for 20 minutes at 55°C. The slides were then washed in 40 mM Tris pH 8.8, 1 mM EDTA, 50 mM NaCl, 0.1 % Tween-20. The slides were scanned at the FITC and Cy-3 channel for 180 sec using a Genetac 1000 microarray slide scanner (Genomic Solutions). The image is shown in Figure 10.

### 6. Results

Figure 10 shows a section of the slide with oligonucleotides printed in the following way:

| | DUPLO | | | | | | | | | |
|---|---|---|---|---|---|---|---|---|---|---|
| | 1 | 3 | 5 | 7 | 9 | 1 | 3 | 5 | 7 | 9 |
| B | cntrl* | 99f41 | 99f42 | 99f43 | 99f44 | cntrl* | 99f41 | 99f42 | 99f43 | 99f44 |
| D | | 99f45 | 99f46 | 99f47 | cntrl* | 99f45 | 99f46 | 99f47 | cntrl* | cntrl* |

| | | | | | | | | | | |
|---|---|---|---|---|---|---|---|---|---|---|
| (cntrl* = controle: to determine what the positions of the oligonucleotides were, a control oligo with an 5' Cy-3 group and an internal amino group was included on the array. | | | | | | | | | | |

Oligonucleotide 99f41 can be extended by ddATP if the ss AFLP marker 1A1 is used. A strong signal is seen on position B3 where the oligo 99f41 is spotted after extension by minisequencing with FAM ddATP. In the duplo the strong signal is also seen. The spots in B1 and D9 are the positive control spots.

### 7. Conclusion

This example demonstrates the specific detection of an AFLP marker by minisequencing on an array containing minisequencing detection primers for 7 different AFLP markers using a single ss AFLP marker fragment.

### EXAMPLE 9. The development of a radioactive AFLP marker detection system on a microchip.

### 1. Minisequencing

The biological material, template preparation, oligo synthesis and the preparation of the slides is as described in Example 9. Minisequencing on ss AFLP fragments was performed according to Syvänen et al. (1990, 1993) with the following modifications. The biotinylated AFLP products were collected on streptavidine-coated magnetic beads (Dynal). Subsequently, minisequencing was preformed on the non biotinylated strand that could be separated from the biotinylated stand by boiling and pipetting off the supematent.
The minisequencing reaction was carried out with 1.25 µl ss reaction of AFLP marker 1A8,1.25 µl reaction of marker 1A11, 1.25 µl reaction of marker 1B1 and 1.25 µl reaction of marker 1G6 in 10 mM TRIS-HCl pH 9.5, 50 mM KCI, 20 mM MgCl₂, 0.02 % Tween-20, 0.42 µl labeled ³³ P ddATP (Amersham), 4.5 units of Thermosequenase and water to a total volume of 40 µl. The reaction was incubated for 20 ' at 55°. The slides were then washed in 40 mM Tris pH 8.8, 1 mM EDTA, 50 mM NaCl, 0.1 % Tween-20. The slides were exposed to Fuji phospho image screens for 16 hours. Patterns were visualized using an Fuji BAS-2000 phosho image analysis system. The image of the minisequencing reaction is shown in Figure 11.

### 2. Results

Figure 11 shows a section of the slide containing the same oligonucleotides as described in Example 9. Oligonucleotides 99f42, 99f44 and 99f45 can be extended with ddATP if respectively ss AFLP reaction 1A8, 1A11 and 1B1 are used. Strong signals are seen at position B5 where oligo 99f42 is spotted, at position B9 where oligo 99f44 is spotted and at position D3 where oligo 99f45 is spotted. In the duplo the strong signals are also seen.
The ss AFLP template 1 G6 had a strong signal on another section of the slide (not shown).

### 3. Conclusion

This example demonstrates the specific detection of 3 AFLP markers by minisequencing on an array containing minisequencing detection primers for 7 different AFLP markers using a mixture of 4 different ss AFLP marker fragments.

## Claims

1. A method for analysing an AFLP reaction mixture for the presence or absence of target restriction fragments in a mixture of restriction fragments, using oligonucleotide sequences that are each specific for only one target restriction fragment, said method comprising the steps of:
a) contacting an AFLP reaction mixture with at least three oligonucleotide sequences under hybridizing conditions, such that when the target restriction fragments are present, hybrids are formed between the target restriction fragments and the oligonucleotide sequences, such that the resulting hybrids have at least one unpaired nucleotide of the target restriction fragment directly adjacent to the 3' end of the oligonucleotide sequence;
b) adding at least one labelled nucleotide, or nucleotide analogue, to the mixture resulting from step a), under conditions suitable for extension of the oligonucleotides, such that when said hybrids are present the oligonucleotides are extended with the labelled nucleotide or nucleotide analogue;
c) detecting the presence or absence of any hybrid with an added labelled nucleotide or nucleotide analogue, and/or of any oligonucleotide sequence with an added labelled nucleotide or nucleotide analogue.

2. Method according to claim 1, wherein each of said oligonucleotides has at least 90% sequence homology to the part of the target restriction fragment for which the oligonucleotide is specific.

3. Method according to any of the preceding claims, wherein during step b) the oligonucleotide sequences are extended by a single labelled nucleotide or nucleotide analogue.

4. Method according to any of the preceding claims, wherein during step a), the AFLP reaction mixture is contacted simultaneously with at least 10, more preferably at least 50, most preferably at least 100 different oligonucleotide sequences, wherein each oligonucleotide sequence is specific for only one target restriction fragment.

5. Method according to any of the preceding claims, wherein the oligonucleotide sequences have a size of about 10 to 100 base pairs, preferably about 20 to 50 base pairs, and optionally contain a "tail", such as a polyT sequence.

6. Method according to any of the preceding claims, wherein the oligonucleotide sequences are immobilized on a solid support, preferably in the form of an array.

7. Method according to any of the preceding claims, wherein the AFLP reaction mixture is obtained/obtainable by restricting DNA with at least one, and preferably with two restriction enzymes, and more preferably with at least one rare cutter restriction enzyme and at least one frequent cutter restriction enzyme, optionally followed by adapter ligation and amplification of the restriction fragments.

8. Method according to any of the preceding claims, wherein after restriction, but prior to step a), the AFLP reaction mixture has been amplified using AFLP primers.

9. Method according to claim 8, wherein the AFLP reaction mixture has been amplified using AFLP primers comprising one or more selective nucleotides at the 3'end.

10. Method according to any of the preceding claims, wherein the target restriction fragment corresponds to an AFLP marker.

11. Method according to claim 1, wherein the same reaction buffers, salt strength, temperature and reaction duration are used for the hybridization of step a) and for the oligonucleotide extension of step b).

12. Method according to any of the preceding claims, wherein step b) is carried out by contacting said hybrids with a reaction mixture comprising a labelled nucleotide or nucleotide analogue that is complementary to only one of A, T, C or G.

## Patentansprüche

1. Verfahren zum Analysieren einer AFLP-Reaktionsmischung auf die Gegenwart oder Abwesenheit von Zielrestriktionsfragmenten in einer Mischung aus Restriktionsfragmenten unter Verwendung von Oligonukleotidsequenzen, welche jeweils nur für ein Zielrestriktionsfragment spezifisch sind, wobei das Verfahren folgende Schritte umfasst,
a) das in Kontakt bringen einer AFLP-Reaktionsmischung mit mindestens drei Oligonukleotidsequenzen unter Hybridisierungsbedingungen, so dass, wenn die Zielrestriktionsfragmente vorliegen, Hybride zwischen den Zielrestriktionsfragmenten und den Oligonukleotidsequenzen gebildet werden, so dass die entstehenden Hybride mindestens ein ungepaartes Nukleotid des Zielrestriktionsfragmentes direkt neben dem 3'-Ende der Oligonukleotidsequenz aufweisen;
b) das Hinzufügen von mindestens einem gekennzeichneten Nukleotid oder einem Nukleotidanalog zu der Mischung resultierend aus Schritt a), unter Bedingungen, die für die Erweiterung der Oligonukleotide geeignet sind, so dass, wenn die Hybride vorliegen, die Oligonukleotide mit dem gekennzeichneten Oligonukleotid oder dem Nukleotidanalog erweitert werden; und
c) das Erkennen der Gegenwart oder Abwesenheit von einem beliebigen Hybrid mit einem hinzugefügten gekennzeichneten Nukleotid oder Nukleotidanalog und/oder einer Oligonukleotidsequenz mit einem hinzugefügten gekennzeichneten Nukleotid oder Nukleotidanalog.

2. Verfahren nach Anspruch 1, wobei jedes der Oligonukleotide mindestens 90% Sequenzhomologie mit dem Teil des Zielrestriktionsfragmentes aufweist, für welchen das Oligonukleotid spezifisch ist.

3. Verfahren nach einem der vorhergehenden Ansprüche, wobei während des Schrittes b) die Oligonukleotidsequenzen durch ein einzelnes gekennzeichnetes Nukleotid oder Nukleotidanalog erweitert werden.

4. Verfahren nach einem der vorhergehenden Ansprüche, wobei während des Schrittes a) die AFLP-Reaktionsmischung gleichzeitig mit mindestens 10, bevorzugter mindestens 50, am bevorzugtesten mindestens 100 unterschiedlichen Oligonukleotidsequenzen in Kontakt gebracht wird, wobei jede Oligonukleotidsequenz nur für ein Zielrestriktionsfragment spezifisch ist.

5. Verfahren nach einem der vorhergehenden Ansprüche, wobei die Oligonukleotidsequenzen eine Größe von etwa 10 bis 100 Basenpaaren, bevorzugt etwa 20 bis 50 Basenpaaren aufweisen und optional einen "Schwanz" enthalten, wie etwa eine Poly-T-Sequenz.

6. Verfahren nach einem der vorhergehenden Ansprüche, wobei die Oligonukleotidsequenzen auf einem festen Träger, bevorzugt in Form eines Arrays, immobilisiert sind.

7. Verfahren nach einem der vorhergehenden Ansprüche, wobei die AFLP-Reaktionsmischung durch Restriktion der DNA mit mindestens einem, und bevorzugt mit zwei Restriktionsenzymen, und bevorzugter mit mindestens einem selten-schneidenden Restriktionsenzym und mindestens einem häufig-schneidenden Restriktionsenzym, optional gefolgt von einer Adapterligation und Amplifikation der Restriktionsfragmente, erhalten wird/zu erhalten ist.

8. Verfahren nach einem der vorhergehenden Ansprüche, wobei nach der Restriktion, jedoch vor Schritt a), die AFLP-Reaktionsmischung unter Verwendung von AFLP-Primern amplifiziert wurde.

9. Verfahren nach Anspruch 8, wobei die AFLP-Reaktionsmischung unter Verwendung von AFLP-Primern amplifiziert wurde, welche ein oder mehrere selektive Nukleotide am 3'-Ende umfassen.

10. Verfahren nach einem der vorhergehenden Ansprüche, wobei das Zielrestriktionsfragment einem AFLP-Marker entspricht.

11. Verfahren nach Anspruch 1, wobei die gleiche/n Reaktionspuffer, Salzstärke, Temperatur und Reaktionsdauer für die Hybridisierung von Schritt a) und für die Oligonukleotiderweiterung von Schritt b) verwendet werden.

12. Verfahren nach einem der vorhergehenden Ansprüche, wobei Schritt b) durch das in Kontakt bringen der Hybride mit einer Reaktionsmischung, welche ein gekennzeichnetes Nukleotid oder Nukleotidanalog umfasst, welches nur zu einem aus A, T, C oder G komplementär ist, durchgeführt wird.

## Revendications

1. Procédé pour analyser un mélange réactionnel d'AFLP pour la présence ou l'absence de fragments de restriction cibles dans un mélange de fragments de restriction, en utilisant des séquences oligonucléotidiques qui sont chacune spécifique d'un seul fragment de restriction cible, ledit procédé comprenant les étapes consistant à :
a) mettre en contact un mélange réactionnel d'AFLP avec au moins trois séquences oligonucléotidiques dans des conditions d'hybridation, de sorte que, lorsque les fragments de restriction cibles sont présents, des hybrides soient formés entre les fragments de restriction cibles et les séquences oligonucléotidiques, de sorte que les hybrides obtenus aient au moins un nucléotide non apparié du fragment de restriction cible directement adjacent à l'extrémité 3' de la séquence oligonucléotidique ;
b) ajouter au moins un nucléotide ou un analogue de nucléotide marqués au mélange résultant de l'étape a), dans des conditions adaptées à l'extension des oligonucléotides, de sorte que, lorsque lesdits hybrides sont présents, les oligonucléotides soient étendus avec le nucléotide ou l'analogue de nucléotide marqués ;
c) détecter la présence ou l'absence d'un hybride quelconque avec un nucléotide ou un analogue de nucléotide marqués ajoutés, et/ou d'une séquence oligonucléotidique quelconque avec un nucléotide ou un analogue de nucléotide marqués ajoutés.

2. Procédé selon la revendication 1, dans lequel chacun desdits oligonucléotides a au moins 90 % d'homologie de séquence avec la partie du fragment de restriction cible dont l'oligonucléotide est spécifique.

3. Procédé selon l'une quelconque des revendications précédentes, dans lequel, pendant l'étape b), les séquences oligonucléotidiques sont étendues par un seul nucléotide ou analogue de nucléotide marqués.

4. Procédé selon l'une quelconque des revendications précédentes, dans lequel, pendant l'étape a), le mélange réactionnel d'AFLP est mis en contact simultanément avec au moins 10, de manière plus préférée au moins 50, de manière préférée entre toutes au moins 100 séquences oligonucléotidiques différentes, chaque séquence oligonucléotidique étant spécifique d'au moins un fragment de restriction cible.

5. Procédé selon l'une quelconque des revendications précédentes, dans lequel les séquences nucléotidiques ont une taille d'environ 10 à 100 paires de bases, de préférence d'environ 20 à 50 paires de bases, et facultativement contiennent une « queue », comme une séquence polyT.

6. Procédé selon l'une quelconque des revendications précédentes, dans lequel les séquences oligonucléotidiques sont immobilisées sur un support solide, de préférence sous forme d'un réseau.

7. Procédé selon l'une quelconque des revendications précédentes, dans lequel le mélange réactionnel d'AFLP est/peut être obtenu par restriction d'un ADN avec au moins une enzyme de restriction, et de préférence deux, et de manière plus préférée avec au moins une enzyme de restriction de césure rare et au moins une enzyme de restriction de césure fréquente, puis facultativement par ligature d'adaptateur et amplification des fragments de restriction.

8. Procédé selon l'une quelconque des revendications précédentes, dans lequel après restriction, mais avant l'étape a), le mélange réactionnel d'AFLP a été amplifié en utilisant des amorces d'AFLP.

9. Procédé selon la revendication 8, dans lequel le mélange réactionnel d'AFLP a été amplifié en utilisant des amorces d'AFLP comprenant un ou plusieurs nucléotides sélectifs à l'extrémité 3'.

10. Procédé selon l'une quelconque des revendications précédentes, dans lequel le fragment de restriction cible correspond à un marqueur AFLP.

11. Procédé selon la revendication 1, dans lequel les mêmes tampons, concentration de sel, température et durée de réaction sont utilisés pour l'hybridation de l'étape a) et pour l'extension d'oligonucléotide de l'étape b).

12. Procédé selon l'une quelconque des revendications précédentes, dans lequel l'étape b) est réalisée par mise en contact desdits hybrides avec un mélange réactionnel comprenant un nucléotide ou un analogue de nucléotide marqués complémentaires d'un seul de A, T, C ou G.
